(19) Europäisches Patentamt
European Patent Office
Office européen des brevets

(11) Publication number: 0 355 049 A2

(12) EUROPEAN PATENT APPLICATION

(21) Application number: 89307454.2

(22) Date of filing: 21.07.89

(51) Int. Cl.4: C07D 249/18 , C07D 401/12 , A01N 43/647

(30) Priority: 18.08.88 GB 8819660
19.06.89 GB 8914041

(43) Date of publication of application: 21.02.90 Bulletin 90/08

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Barton, John Edward Duncan
96 Kendrick Road
Reading Berkshire(GB)

(74) Representative: Hardman, Carol Pauline et al
Imperial Chemical Industries PLC Legal Department: Patents PO Box 6 Bessemer Road
Welwyn Garden City Herts, AL7 1HD(GB)

# (54) Heterocyclic compounds.

(57) A herbicidal compound of formula (I):

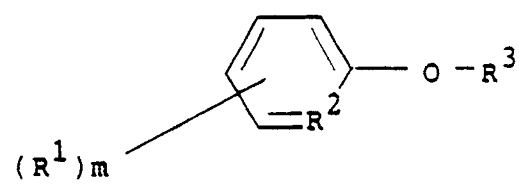

(I)

in which $R^1$ is independently selected from H, CN, $NO_2$, halogen, lower alkyl, lower haloalkyl; m is an integer of from 1 to 4; $R^2$ is N or $CR^1$ where $R^1$ is as defined above; $R^3$ is a group of formula (a), (b) or (c):

EP 0 355 049 A2

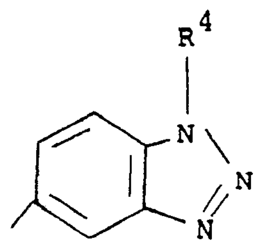

(a)

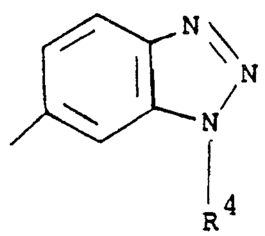

(b)

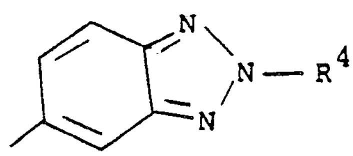

(c)

or N-oxide or quaternary salt derivatives thereof wherein $R^4$ is a specified organic group. Processes for the preparation of these compounds and compositions containing them are also described.

# HETEROCYCLIC COMPOUNDS

The present invention relates to novel substituted benzotriazole derivatives, processes for their preparation, their use as herbicides and herbicidal compositions containing them.

European Patent No. 178,708 A describes certain benzheterocyclyl-phenyl ether derivatives which have herbicidal activity.

According to the present invention there is provided a compound of formula (I):

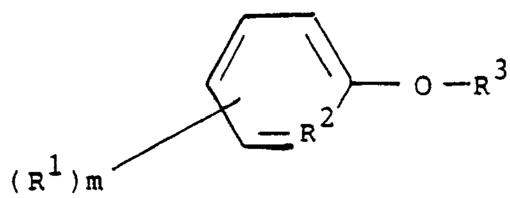

(I)

in which $R^1$ is independently selected from H, CN, $NO_2$, halogen, lower alkyl, lower haloalkyl; m is an integer of from 1 to 4; $R^2$ is N or $CR^1$ where $R^1$ is as defined above; $R^3$ is a group of formula (a), (b) or (c):

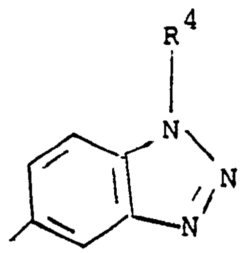

(a)

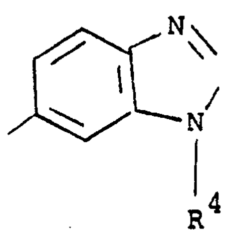

(b)

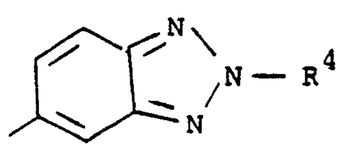

(c)

or N-oxide or quaternary salt derivatives thereof wherein $R^4$ is a group

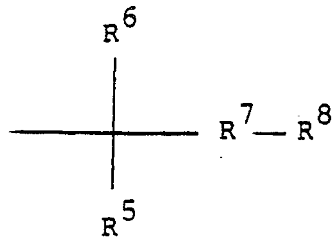

where $R^5$, $R^6$ are selected independently from H, lower alkyl, halogen or $R^5$ and $R^6$ together form $=CH_2$ or $R^5$, $R^6$ and the carbon atom to which they are attached together form a $C_{3-6}$ cycloalkyl ring;
$R^7$ is $(CH_2)_n$,

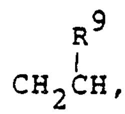

$CH=CH$,
$\overset{O}{\overset{\|}{C}}CH_2$, $CHOHCH_2$ or $CHOR^{10}CH_2$ where $R^9$ is lower alkyl and $R^{10}$ is lower alkyl or phenyl; and n is 0, 1 or 2;
$R^8$ is $COOR^{11}$, OH, $OR^{10}$,

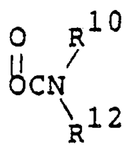 , 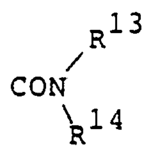 ,

$COSR^{11}$ CHO, CN, $CH=NOR^{12}$,
$\overset{O}{\overset{\|}{P}}(OR^{12})_2$, $OSO_3H$, $SO_3H$; where $R^{10}$ is as defined hereinbefore;
$R^{11}$ is hydrogen, optionally substituted lower alkyl, optionally substituted lower alkenyl, optionally substituted lower alkynyl, $[(CH_2)_2O]_p(CH_2)_q$ $OR^{10}$, $-N=C(R^{10})_2$, optionally subtituted phenyl, optionally substituted benzyl, optionally substituted cycloalkyl; p is 0, 1 or 2; q is an integer from 2 to 5 inclusive;
$R^{12}$ is H or lower alkyl;
$R^{13}$ and $R^{14}$ are selected independently from H, lower alkyl, lower cycloalkyl, alkenyl, alkynyl, $SO_2R^{10}$, $N(R^{12})_2$, $^+N(R^{10})_3$; additionally $R^{14}$ may be an optionally hydroxy- or phenyl-substituted alkyl radical of 1 to 4 carbon atoms, a phenyl or chlorophenyl radical, an alkoxy radical of 1 to 4 carbon atoms, or a group $-NR^{15}R^{16}$ wherein $R^{15}$ is hydrogen or alkyl of 1 to 4 carbon atoms and $R^{16}$ is hydrogen, alkyl of 1 to 4 carbon atoms, phenyl, or chlorophenyl, or the group $-NR^{13}R^{14}$ constitutes a 5 or 6-membered heterocyclic ring or alkyl quaternary derivatives; and, in the case of compounds wherein $R^8$ is a carboxyl group, salts thereof.

Some of the compounds can exist in optical isomeric forms and the present invention covers all these forms and mixtures thereof in all proportions including racemic mixtures.

Suitable optional substituents for the alkyl, alkenyl and alkynyl group of $R^{11}$ are one or more halogen atoms such as fluorine, chlorine, bromine or iodine, hydroxy and C1-C4 alkoxy. Suitable optional substituents for the phenyl, benzyl and cycloalkyl groups are one or more halogen atoms such as fluorine, chlorine, bromine or iodine or methyl groups.

Suitable halo groups $R^1$, $R^5$ and $R^6$ include fluorine, chlorine and bromine.

Suitable haloalkyl groups $R^1$, $R^5$ and $R^6$ include $C_{1-6}$ alkyl groups substituted with up to 6 halogen atoms selected from fluorine, chlorine and bromine. A particular haloalkyl group is trifluoromethyl. Suitable heterocyclic rings formed from $NR^{13}$ $R^{14}$ are pyrrolidine, piperidine and morpholine.

Suitable salts of compounds of formula (I) are those formed with agriculturally acceptable cations, for example sodium, potassium or quaternary ammonium salts.

Examples of quaternary ammonium ions are ions of formula $NR^{17}R^{18}R^{19}R^{20}$ where $R^{17}$, $R^{18}$, $R^{19}$ and $R^{20}$ are independently selected from hydrogen or $C_{1-10}$ alkyl optionally substituted with for example hydroxy. Suitably where any of $R^{17}$, $R^{18}$, $R^{19}$ and $R^{20}$ are optionally substituted alkyl, they contain from 1 to 4 carbon atoms.

Particular examples of $R^{17}$, $R^{18}$, $R^{19}$ and $R^{20}$, are hydrogen, ethyl and 2-hydroxyethyl.

As used herein the term "lower alkyl" includes groups containing from 1 to 10 carbon atoms, preferably from 1 to 6 carbon atoms. Similarly the terms "lower alkenyl" and "lower alkynyl" includes groups containing from 2 to 10 carbon atoms, preferably from 2 to 6 carbon atoms.

Preferably $R^1$ is selected from hydrogen, chlorine, fluorine or trifluoromethyl wherein at least one of the $R^1$ groups is other than hydrogen and at least one of the $R^1$ groups is trifluoromethyl.

Preferably $R^5$ and $R^6$ are H, lower alkyl or halogen, in particular hydrogen, methyl or fluoro.

A preferred example of the group $R^2$ is C-Cl.

Preferably $R^3$ is the group (b) as hereinbefore defined.

Preferably $R^4$ is

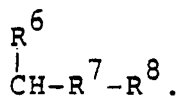

Preferably $R^7$ is $(CH_2)_n$ and n is 0 or 1, in particular 0.

Suitable groups $R^8$ are alkoxycarbonyl groups wherein the alkoxy group is straight or branched and contains up to 6 carbon atoms. In particular the group $R^8$ is $C_{1-4}$ alkoxycarbonyl, preferably ethoxycarbonyl.

A suitable sub-group of compounds of formula (I) are compounds where $(R^1)_m$ is independently selected from hydrogen, halo, nitro, cyano or haloalkyl, and m is an integer of from 1 to 4; $R^2$ is C-$R^1$ where $R^1$ is hydrogen, halo, nitro, cyano or haloalkyl; $R^3$ is a group of formula (a), (b) or (c) as hereinbefore defined; $R^4$ is

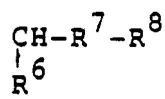

wherein $R^6$ is H or $C_{1-4}$ alkyl, $R^7$ is $(CH_2)n$ where n is O or 1, preferably O and $R^8$ is a cyano group, a carboxyl group, an alkoxycarbonyl group or carboxamido group $CONR^{13}R^{14}$ as hereinbefore defined.

A further sub-group of compounds of formula (I) are compounds where $(R^1)m$ is independently selected from hydrogen, halo, nitro, cyano or haloalkyl and m is an integer of from 1 to 4; $R^2$ is C-$R^1$ where $R^1$ is hydrogen, halo, nitro, cyano or haloalkyl; $R^3$ is a group of formula (a), (b) or (c) as hereinbefore defined; $R^4$ is

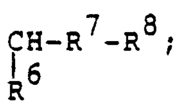

wherein $R^6$ is H, $C_{1-4}$ alkyl or halo, $R^7$ is $(CH_2)n$ where n is 0 or 1, preferably 0 and $R^8$ is a group $COOR^{11}$ where $R^{11}$ is as hereinbefore defined.

Another sub-group of suitable compounds of formula (I) are compounds where $(R^1)_m$ is independently selected from hydrogen, halo, nitro, cyano or haloalkyl and m is an integer of from 1 to 4; $R^2$ is C-$R^1$ where $R^1$ is hydrogen, halo, nitro, cyano or haloalkyl; $R^3$ is a group of formula (b) or (c) as hereinbefore defined; $R^4$ is

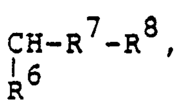

$R^6$ is H or $C_{1-4}$ alkyl or halo, $R^7$ is $(CH_2)n$ where n is 0, 1 or 2, preferably 0; $R^8$ is a group $COOR^{11}$ where $R^{11}$ is as hereinbefore defined, and particularly $C_{1-4}$ alkyl, preferably ethyl.

Particular examples of compounds according to the invention are listed in Tables I, II and III.

TABLE I

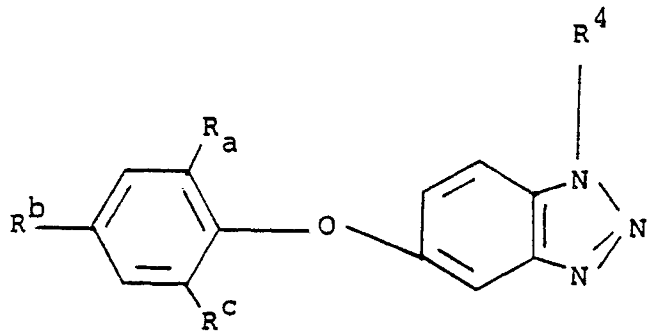

| Compound No | $R^a$ | $R^b$ | $R^c$ | $R^4$ | Characterising Data (NMR unless otherwise stated) |
|---|---|---|---|---|---|
| 1 | Cl | Cl | H | $CH_3$<br>$CHCOOC_2H_5$ | $CDCl_3$ δ7.51(m)3H;7.26(m)2H; 6.96(d)1H; 5.66(q)1H; 4.21 (q)2H; 2.02(d)3H;1.23 (t)3H. |
| 4 | Cl | Cl | H | $CH_2COOC_2H_5$ | $CDCl_3$ δ7.49(m)3H; 7.20-7.35 (m)2H; 6.95(d)1H; 5.40(s) 2H; 4.28(q)2H; 1.29(t)3H. |
| 7 | Cl | $CF_3$ | H | $CH_3$<br>$CHCOOC_2H_5$ | $CDCl_3$ δ7.79(s)1H; 7.60(m) 2H; 7.46(d)1H; 7.3(dd) 1H; 7.00(d)1H; 5.7(q)1H; 4.24(q)2H; 2.01(d) 3H; 1.24(t)3H. |
| 10 | Cl | $CF_3$ | F | $CH_3$<br>$CHCOOC_2H_5$ | $CDCl_3$ δ7.62(s)1H; 7.54(d) 1H; 7.44(dd)1H; 7.35(dd) 1H; 7.29(s)1H; 5.68(q) 1H; 4.21(q)2H; 2.00(d)3H; 1.21 (t)3H. |

TABLE I (Contd.)

| Compound No | $R^a$ | $R^b$ | $R^c$ | $R^4$ | Characterising Data (NMR unless otherwise stated) |
|---|---|---|---|---|---|
| 13 | Cl | $CF_3$ | F | $CH_3$ \| CHCOOH | m.p248.5-249°C |
| 16 | Cl | $CF_3$ | F | $CH_2COOC_2H_5$ | $CDCl_3$ δ7.62(s)1H; 7.35-7.5 (m)4H; 5.40(s)2H; 4.27(q) 2H; 1.30(t)3H. |
| 19 | Cl | $CF_3$ | F | F \| $CHCOOC_2H_5$ | $CDCl_3$ 7.66(d)1H δ7.63(s)1H; 7.4-7.5(m)2H; 7.35(s)1H; 7.21+7.03(d)1H; 4.39(m)2H; 1.30(t)3H. |
| 22 | Cl | $CF_3$ | F | F \| $CHCOOC_4H_9$ | $CDCl_3$ 7.66(d)1H δ7.64(s)1H; 7.44(m) 2H; 7.35(s)1H; 7.21+7.03(d)1H; 4.30(t)2H; 59(m)2H; 1.23(m)2H; 0.85(t)3H. |
| 25 | Cl | $CF_3$ | F | F \| CHCOOH | m.p 177-177.8°C |
| 28 | Cl | $CF_3$ | F | $C_2H_5$ \| $CHCOOC_2H_5$ | $CDCl_3$ δ7.63(s)1H; 7.60(d) 1H;7.45(dd)1H; 7.35(dd)1H; 7.29(d)1H; 5.47(dd)1H; 4.22 (q)2H; 2.35-2.6(m)2H; 1.24 (t)3H; 0.91(t)3H. |

TABLE I (Contd.)

| Compound No | $R^a$ | $R^b$ | $R^c$ | $R^4$ | Characterising Data (NMR unless otherwise stated) |
|---|---|---|---|---|---|
| 31 | H | $CF_3$ | H | $CH_3$<br>\|<br>$CHCOOC_2H_5$ | $CDCl_3$ δ7.70(d)1H; 7.60(m) 3H; 7.29(d)1H; 7.09(d)2H; 5.70(q)1H; 4.24(q)2H; 2.01 (d)3H; 1.24(t)3H. |
| 34 | H | $CF_3$ | H | $CH_3$<br>\|<br>$C-COOC_2H_5$<br>\|<br>$CH_3$ | $CDCl_3$ δ7.69(d)1H; 7.60(d) 2H; 7.44(d)1H; 7.22(dd)1H; 7.07(d)2H; 4.21(q)2H; 2.10 (s)6H; 1.16(t)3H. |
| 43 | H | $CF_3$ | H | $CH_3$<br>\|<br>$CH(CH_2)COOC_2H_5$ | $CDCl_3$ δ7.69(d)1H;7.59(m) 3H; 7.25(dd)1H; 7.05(d)2H; 5.0-5.15(m)1H; 4.10(q)2H; 2.6-2.42(m)1H; 2.4-2.29(m) 1H; 2.1-2.3(m)2H; 1.77(d) 3H; 1.21(t)3H. |
| 44 | Cl | $CF_3$ | F | $(CH_2)_2COOC_2H_5$ | $CDCl_3$ δ7.62(m)2H; 7.43(dd) 1H; 7.36(dd)1H; 7.24(d)1H; 4.89(t)2H; 4.11(t)2H; 3.09 (t)2H; 1.20(t)3H. |

TABLE I (Contd.)

| Compound No | $R^a$ | $R^b$ | $R^c$ | $R^4$ | Characterising Data (NMR unless otherwise stated) |
|---|---|---|---|---|---|
| 45 | Cl | $CF_3$ | F | $CH_3$<br>$CHCH_2COOC_2H_5$ | $CDCl_3$ δ7.60(m)2H; 7.43(dd)1H; 7.36(dd)1H; 7.25(d)1H; 5.31(m)1H; 4.02(m)2H; 3.30(dd)1H; 3.01(dd)1H; 1.75(d)3H; 1.12(t)3H. |
| 46 | Cl | $CF_3$ | F | $CH_3$<br>$CH_2CHCOOC_2H_5$ | $CDCl_3$ δ7.62(s)1H; 7.58(d)1H; 7.43(dd)1H; 7.35(dd)1H; 7.24(s)1H; 4.87(dd)1H; 4.65(dd)1H; 4.06(m)2H; 3.26(m)1H; 1.28(d)3H; 1.12(t)3H. |
| 47 | Cl | $CF_3$ | F | $CH_3$<br>$CH(CH_2)_2COOC_2H_5$ | $CDCl_3$ 7.55(s)1H; 7.49(d)1H; 7.36(dd)1H; 7.26(dd)1H; 7.18(d)1H; 4.90-5.01(m)1H; 3.99(q)2H; 2.00-2.50(m)4H; 1.66(d)3H; 1.14(t)3H. |
| 65 | H | $CF_3$ | $NO_2$ | $CH_3$<br>$CHCOOC_2H_5$ | $CDCl_3$ δ8.29(d)1H; δ7.74(dd+d)2H; δ7.64(d)1H; 7.32(dd)1H; δ7.1(d)1H; δ5.73(q)1H; δ4.25(q)2H; δ2.07(d)3H; δ1.25(t)3H. |

TABLE I (Contd.)

| Compound No | $R^a$ | $R^b$ | $R^c$ | $R^4$ | Characterising Data (NMR unless otherwise stated) |
|---|---|---|---|---|---|
| 66 | Cl | $CF_3$ | $NO_2$ | $CH(CH_3)COOC_2H_5$ | $CDCl_3$ δ8.21(d)1H; δ8.05(d) 1H; δ7.57(d)1H; δ7.34(dd) 1H; δ7.2(d)1H; δ5.68(q)1H; δ4.22(q)2H; δ2.0(d)3H; δ1.23(t)3H. |
| 67 | F | $CF_3$ | F | $CH(CH_3)COOC_2H_5$ | $CDCl_3$ δ7.5-7.62(m)1H; δ7.3-7.4(m)4H; δ5.68(q)1H; δ4.2(q)2H; δ2.0(d)3H; δ1.25(t)3H. |
| 68 | Cl | $CF_3$ | Cl | $CH(CH_3)COOC_2H_5$ | $CDCl_3$ δ7.72(s)2H; δ7.56(d) 1H; δ7.34(dd)1H; δ7.15(d) 1H; δ5.68(q)1H; δ4.22(q)2H; δ2.0(d)3H; γ1.22(t)3H. |
| 75 | H | $CF_3$ | CN | $CH(CH_3)COOC_2H_5$ | $CDCl_3$ δ8.18(d)1H; δ7.98(d) 1H; δ7.85(dd)1H; δ7.44(d) 1H; δ7.28(dd)1H; δ6.95(d) 1H; δ5.68(q)1H; δ4.22(q)2H; δ2.04(d)3H; δ1.25(t)3H. |
| 76 | Cl | $CF_3$ | CN | $CH(CH_3)COOC_2H_5$ | $CDCl_3$ δ8.07(d)1H; δ8.02(d) 1H; δ7.92(d)1H; δ7.0(dd)1H; δ6.94(d)1H; δ5.5(q)1H; δ4.15(q)2H; δ1.99(d)3H; δ1.18(t)3H. |

TABLE II

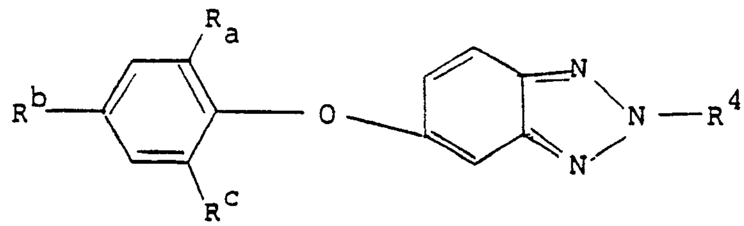

| Compound No | $R^a$ | $R^b$ | $R^c$ | $R^4$ | Characterising Data (NMR unless otherwise stated) |
|---|---|---|---|---|---|
| 2 | Cl | Cl | H | $CH(CH_3)COOC_2H_5$ | $CDCl_3$ δ7.87(d)1H; 7.50(s) 1H; 7.43(m)2H; 7.12(s)1H; 7.01(d)1H; 5.63(q)1H; 4.23 (q)2H; 2.03(d)3H; 1.24(t) 3H. |
| 5 | Cl | Cl | H | $CH_2COOC_2H_5$ | $CDCl_3$ δ7.78(d)1H; 7.40(s) 1H; 7.12(m)2H; 7.01(s)1H; 6.90(d)1H; 5.39(s)2H; 4.15 (q)2H; 1.08(t)3H. |
| 8 | Cl | $CF_3$ | H | $CH(CH_3)COOC_2H_5$ | $CDCl_3$ δ7.92(d)1H; 7.78(s) 1H; 7.49(dd)1H; 7.3(s)1H; 7.21(dd)1H; 7.08(d)1H; 5.66 (q)1H; 4.24(q)2H; 2.04(d) 3H; 1.25(t)3H. |
| 11 | Cl | $CF_3$ | F | $CH(CH_3)COOC_2H_5$ | $CDCl_3$ δ7.91(d)1H; 7.61(s) 1H; 7.45(dd)1H; 7.30(dd) 1H; 6.98(s)1H; 5.64(q)1H; 4.22(q)2H; 2.00(d)3H; 1.22 (t)3H. |

TABLE II (Contd.)

| Compound No | $R^a$ | $R^b$ | $R^c$ | $R^4$ | Characterising Data (NMR unless otherwise stated) |
|---|---|---|---|---|---|
| 14 | Cl | $CF_3$ | F | $CH_3$ <br> \| <br> CHCOOH | m.p. 168-169.3°C |
| 17 | Cl | $CF_3$ | F | $CH_2COOC_2H_5$ | $CDCl_3$ δ7.90(d)1H; 7.62(s) 1H; 7.44(dd)1H; 7.31(dd)1H; 6.97(s)1H; 5.46(s)2H; 4.27 (q)2H; 1.30(t)3H. |
| 20 | Cl | $CF_3$ | F | F <br> \| <br> $CHCOOC_2H_5$ | $CDCl_3$ δ7.95(d)1H; 7.64(s) 1H; 7.46(dd)1H; 7.40(dd)1H; 6.92(s)1H; 6.81+7.01(d)1H; 4.40(m)2H; 1.33(t)3H. |
| 23 | Cl | $CF_3$ | F | F <br> \| <br> $CHCOOC_4H_9$ | $CDCl_3$ δ7.93(d)1H; 7.63(s) 1H; 7.46(dd)1H; 7.40(dd)1H; 7.01+6.82(d)1H; 6.91(s)1H; 4.35(t)2H; 1.65(m)2H; 1.3 (m)2H; 0.88(t)3H. |
| 26 | Cl | $CF_3$ | F | F <br> \| <br> CHCOOH | m.p. 135.8-137°C |
| 29 | Cl | $CF_3$ | F | $C_2H_5$ <br> \| <br> $CHCOOC_2H_5$ | $CDCl_3$ δ7.90(d)1H; 7.62(s) 1H; 7.44(dd)1H; 7.31(dd)1H; 6.97(d)1H; 5.40(dd)1H; 4.21 (m)2H; 2.37-2.65(m)2H; 1.23 (t)3H; 0.94(t)3H. |

TABLE II (Contd.)

| Compound No | $R^a$ | $R^b$ | $R^c$ | $R^4$ | Characterising Data (NMR unless otherwise stated) |
|---|---|---|---|---|---|
| 32 | H | $CF_3$ | H | $CH_3$<br>$CHCOOC_2H_5$ | $CDCl_3$ δ7.91(d)1H; 7.60(d) 2H; 7.40(s)1H; 7.19(dd)1H; 7.11(d)2H; 5.66(q)1H; 4.23 (q)2H; 2.01(d)3H; 1.25(t) 3H. |
| 35 | H | $CF_3$ | H | $CH_3$<br>$C-COOC_2CH_5$<br>$CH_3$ | $CDCl_3$ δ7.90(d)1H; 7.60(d) 2H; 7.39(d)1H; 7.18(dd)1H; 7.1(d)2H; 4.2(q)2H; 2.10(s) 6H; 1.20(t)3H. |
| 42 | Cl | $CF_3$ | H | $CH_3$<br>$CHCOOH$ | DMSO δ7.99(d)1H; 7.90(s) 1H; 7.60(d)1H; 7.45(s)1H; 7.21(dd)1H; 7.15(d)1H; 5.75 (q)1H; 1.82(d)3H. |
| 48 | H | $CF_3$ | H | $CH_3$<br>$CH(CH_2)_2COOC_2H_5$ | $CDCl_3$ δ7.89(d)1H; 7.61(d) 2H; 7.40(d)1H; 7.15(dd)1H; 7.09(d)2H; 4.9-5.15(m)1H; 4.10(q)2H; 2.22-2.58(m)2H; 2.19(d)2H; 1.75(d)3H; 1.21(t)3H. |

TABLE II (Contd.)

| Compound No | $R^a$ | $R^b$ | $R^c$ | $R^4$ | Characterising Data (NMR unless otherwise stated) |
|---|---|---|---|---|---|
| 49 | Cl | $CF_3$ | F | $(CH_2)_2COOC_2H_5$ | $CDCl_3$ 7.86(d)1H; 7.61(s)1H; 7.44(dd)1H; 7.28(dd)1H; 6.95(s)1H; 5.96(t)2H; 4.16 (q)2H; 3.12(t)2H; 1.24 (t)3H. |
| 50 | Cl | $CF_3$ | F | $CH(CH_3)CH_2COOC_2H_5$ | $CDCl_3$ δ7.87(d)1H; 7.61(s) 1H; 7.42(dd)1H; 7.28(dd)1H; 6.94(s)1H; 5.40(m)1H; 4.09 (q)2H; 3.26(dd)1H; 2.92(dd) 1H; 1.70(d)3H; 1.16(t)3H. |
| 51 | Cl | $CF_3$ | F | $CH_2CH(CH_3)COOC_2H_5$ | $CDCl_3$ δ7.85(d)1H; 7.61(s) 1H; 7.42(dd)1H; 7.26(dd)1H; 6.94(d)1H; 4.99(dd)1H; 4.70 (dd)1H; 4.15(q)2H; 3.31(m) 1H; 1.20(m)6H. |
| 52 | Cl | $CF_3$ | F | $CH(CH_3)(CH_2)_2COOC_2H_5$ | $CDCl_3$ δ7.87(d)1H; 7.61(s) 1H; 7.42(dd)1H; 7.27(dd)1H; 6.95(d)1H; 4.94-5.09(m)1H; 4.07(q)2H; 2.1-2.5(m)4H; 1.69(d)3H; 1.21(t)3H. |

TABLE III

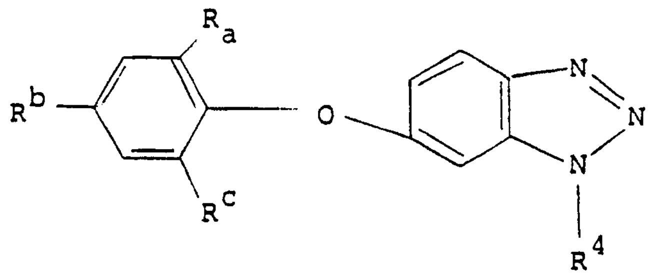

| Compound No | $R^a$ | $R^b$ | $R^c$ | $R^4$ | Characterising Data (NMR unless otherwise stated) |
|---|---|---|---|---|---|
| 3 | Cl | Cl | H | $CH(CH_3)COOC_2H_5$ | $CDCl_3$ δ7.96(d)1H; 7.43(s) 1H; 7.17(dd)1H; 7.00(dd)1H; 6.92(d)1H; 6.84(s)1H; 5.46 (q)1H; 4.11(q)2H; 1.92(d) 3H; 1.11(t)3H. |
| 6 | Cl | Cl | H | $CH_2COOC_2H_5$ | $CDCl_3$ δ8.04(d)1H; 7.51(d) 1H; 7.26(dd)1H; 7.11(dd)1H; 7.01(d)1H; 6.82(d)1H; 5.31 (s)2H; 4.22(q)2H; 1.25(t) 3H. |
| 9 | Cl | $CF_3$ | H | $CH(CH_3)COOC_2H_5$ | $CDCl_3$ δ8.09(d)1H; 7.79(s) 1H; 7.50(d)1H; 7.0-7.15(m) 3H; 5.59(q)1H; 4.20(q)2H; 2.00(d)3H; 1.20(t)3H. |
| 12 | Cl | $CF_3$ | F | $CH(CH_3)COOC_2H_5$ | $CDCl_3$ δ8.03(d)1H; 7.62(s) 1H; 7.45(dd)1H; 7.08(dd) 1H; 6.9(s)1H; 5.54(q)1H; 4.17(q)2H; 1.99(d)3H; 1.16 (t)3H. |

TABLE III (Contd.)

| Compound No | $R^a$ | $R^b$ | $R^c$ | $R^4$ | Characterising Data (NMR unless otherwise stated) |
|---|---|---|---|---|---|
| 15 | Cl | $CF_3$ | F | $CH(CH_3)COOH$ | m.p. 173-173.9°C |
| 18 | Cl | $CF_3$ | F | $CH_2COOC_2H_5$ | $CDCl_3$ δ8.05(d)1H; 7.63(s) 1H; 7.45(dd)1H; 7.10(dd) 1H; 6.80(s)1H; 5.31(s)2H; 4.23(q)2H; 1.22(t)3H. |
| 21 | Cl | $CF_3$ | F | $CHFCOOC_2H_5$ | $CDCl_3$ δ8.10(d)1H; 7.65(s) 1H; 7.47(dd)1H; 7.19(d) 0.5H; 7.15(s)1H; 6.96(s) 1.5H; 4.35(q)2H; 1.24(t) 3H. |
| 24 | Cl | $CF_3$ | F | $CHFCOOC_4H_9$ | $CDCl_3$ δ8.10(d)1H; 7.65(s) 1H; 7.47(dd)1H; 7.15(dd) 1H; 6.99(d)1H; 7.16+6.98 (d)1H; 4.27(t)2H; 1.57(m) 2H; 1.22(m)2H; 0.85(t)3m. |
| 27 | Cl | $CF_3$ | F | $CHFCOOH$ | m.p. 150.5-151.6°C |
| 30 | Cl | $CF_3$ | F | $CH(C_2H_5)COOC_2H_5$ | $CDCl_3$ δ8.04(d)1H; 7.62(s) 1H; 7.44(dd)1H; 7.06(dd) 1H; 6.94(d)1H; 5.36(dd)1H; 4.19(q)2H; 2.3-2.51(m)2H; 1.18(t)3H; 0.90(t)3H. |

TABLE III (Contd.)

| Compound No | $R^a$ | $R^b$ | $R^c$ | $R^4$ | Characterising Data (NMR unless otherwise stated) |
|---|---|---|---|---|---|
| 33 | H | $CF_3$ | H | $CH_3$ \| $CHCOOC_2H_5$ | $CDCl_3$ δ8.08(d)1H; 7.62(d) 2H; 7.10(m)4H; 5.60(q)1H; 4.20(q)2H; 1.99(d)3H; 1.20 (t)3H. |
| 36 | H | $CF_3$ | H | $CH_3$ \| $C-COOC_2H_5$ \| $CH_3$ | $CDCl_3$ δ8.08(d)1H; 7.60(d) 2H; 7.10(m)1H; 7.07(d)2H; 7.01(d)1H; 4.16(q)2H; 2.06 (s)6H; 1.13(t)3H. |
| 37 | Cl | $CF_3$ | F | $CH_3$ \| $CHCOOC_4H_9$ | $CDCl_3$ δ8.03(d)1H; 7.62(s) 1H; 7.45(dd)1H; 7.06(dd) 1H; 6.89(s)1H; 5.53(q)1H; 4.11(t)2H; 2.00(d)3H; 1.49 (m)2H; 1.16(m)2H; 0.81(t) 3H. |
| 38 | Cl | $CF_3$ | F | $CH_3$ \| $CHCONHSO_2CH_3$ | mass spec. M+ 480 |
| 39 | Cl | $CF_3$ | F | $CH_3$ \| $CHCH_2OH$ | $CDCl_3$ δ7.90(d)1H; 7.65(s) 1H; 7.45(dd)1H; 7.05(dd) 1H; 6.89(s)1H; 4.79(m)1H; 4.22(m)1H; 4.10(m)1H; 2.79(t)1H; 1.63(t)3H. |

TABLE III (Contd.)

| Compound No | $R^a$ | $R^b$ | $R^c$ | $R^4$ | Characterising Data (NMR unless otherwise stated) |
|---|---|---|---|---|---|
| 40 | Cl | $CF_3$ | F | $CH_3$<br>$CHCONHN(CH_3)_2$ | mass spec. M+ 445 |
| 41 | Cl | $CF_3$ | F | $CH_3$ $^+$ $I^-$<br>$CHCONHN(CH_3)_3$ | mass spec. M+ 460 |
| 53 | H | $CF_3$ | H | $CH_3$<br>$CH(CH_2)_2COOC_2H_5$ | $CDCl_3$ δ8.03(d)1H; 7.61(d) 2H; 7.1(m)4H; 4.82-5.0(m) 1H; 4.03(q)2H; 2.4-2.6(m) 1H; 2.20-2.39(m)1H; 2.20 (dd)2H; 1.72(d)3H; 1.20(t) 3H. |
| 54 | Cl | $CF_3$ | F | $CH_3$<br>CH-COOtBu | $CDCl_3$ δ8.02(d)1H; 7.61(s) 1H; 7.43(dd)1H; 7.08(dd) 1H; 6.81(d)1H; 5.42(q)1H; 1.94(d)3H; 1.32(s)9H. |
| 55 | Cl | $CF_3$ | F | $CH_3$<br>$CHCONH_2$ | m.p. 179.1-180.6°C. |
| 56 | Cl | $CF_3$ | F | $CH_3$<br>$CHCON(C_2H_5)_2$ | $CDCl_3$ δ7.99(d)1H; 7.61(s) 1H; 7.42(dd)1H; 7.19(d)1H; 7.05(dd)1H; 5.99(q)1H; 3.15-3.50(m)4H; 1.75(d)3H; 1.00(t)3H; 0.89(t)3H. |

TABLE III (Contd.)

| Compound No | $R^a$ | $R^b$ | $R^c$ | $R^4$ | Characterising Data (NMR unless otherwise stated) |
|---|---|---|---|---|---|
| 57 | Cl | $CF_3$ | F | $CH(CH_3)CONHC_2H_5$ | $CDCl_3$ δ8.05(d)1H; 7.64(s) 1H; 7.45(dd)1H; 7.15(dd) 1H; 6.81(d)1H; 6.0-6.15(s) 1H; 5.29(q)1H; 3.10-3.35 (m)2H; 1.95(d)3H; 1.04(t) 3H. |
| 58 | Cl | $CF_3$ | F | $(CH_2)_2COOC_2H_5$ | $CDCl_3$ δ8.00(d)1H; 7.65(s)1H; 7.46(dd) 1H; 7.10(dd)1H; 6.91(d)1H; 4.79(t)2H; 4.09(q)2H; 3.05 (t)2H; 1.19(t)3H. |
| 59 | Cl | $CF_3$ | F | $CH(CH_3)CH_2COOC_2H_5$ | $CDCl_3$ δ8.00(d)1H; 7.64(s) 1H; 7.46(dd)1H; 7.07(dd)1H; 6.90(d)1H; 5.15(m)1H; 4.02(q)2H; 3.26(dd)1H; 3.00 (dd)1H; 1.70(d)3H; 1.11(t) 3H. |
| 60 | Cl | $CF_3$ | F | $CH_2CH(CH_3)COOC_2H_5$ | $CDCl_3$ δ7.99(d)1H; 7.63(s) 1H; 7.45(dd)1H; 7.08(dd) 1H; 6.89(s)1H; 4.76(dd)1H; 4.56(dd)1H; 4.02(m)2H; 3.22 (m)1H; 1.24(d)3H; 1.12(t)3H |

TABLE III (Contd.)

| Compound No | $R^a$ | $R^b$ | $R^c$ | $R^4$ | Characterising Data (NMR unless otherwise stated) |
|---|---|---|---|---|---|
| 61 | Cl | $CF_3$ | F | $CH_3$ \| *CHCOOH *R Form | m.p. 156.1-159.0°C. |
| 62 | Cl | $CF_3$ | F | $CH_3$ \| *CHCOOH *S Form | m.p. 155.9-160.0°C. |
| 63 | Cl | $CF_3$ | F | $CH_3$ \| *$CHCOOC_2H_5$ R Form | $CDCl_3$ δ8.04(d)1H; 7.63(s) 1H; 7.44(dd)1H; 7.06(dd) 1H; 6.88(d)1H; 5.53(q)1H; 4.16(q)2H; 1.98(d)3H; 1.16 (t)3H. |
| 64 | Cl | $CF_3$ | F | $CH_3$ \| *$CHCOOC_2H_5$ S Form | $CDCl_3$ δ8.03(d)1H; 7.63(s) 1H; 7.44(dd)1H; 7.06(dd) 1H; 6.88(d)1H; 5.53(q)1H; 4.16(q)2H; 1.97(d)3H; 1.15 (t)3H; |
| 69 | H | $CF_3$ | $NO_2$ | $CH_3$ \| $CHCOOC_2H_5$ | $CDCl_3$ δ8.28(d)1H; δ8.13(d) 1H; δ7.77(dd)1H; δ7.30(d) 1H; δ7.14(dd+d)2H; δ5.65(q) 1H; δ4.2(q)2H; δ2.02(d)3H; δ1.25(t)3H. |

TABLE III (Contd.)

| Compound No | $R^a$ | $R^b$ | $R^c$ | $R^4$ | Characterising Data (NMR unless otherwise stated) |
|---|---|---|---|---|---|
| 70 | Cl | $CF_3$ | $NO_2$ | $CH_3$<br>$CHCOOC_2H_5$ | $CDCl_3$ δ8.20(d)1H; 8.04(m) 2H; 7.0(dd)1H; δ6.89(d)1H; δ5.55(q)1H; δ4.18(q)2H; δ2.0(d)3H; δ1.15(t)3H. |
| 71 | F | $CF_3$ | F | $CH_3$<br>$CHCOOC_2H_5$ | $CDCl_3$ δ8.0(d)1H; δ7.36(d) 2H; 7.10(dd)1H; δ6.97(d) 1H; δ5.56(q)1H; δ4.18(q) 2H; δ2.0(d)3H; δ1.18(t)3H. |
| 72 | Cl | $CF_3$ | Cl | $CH_3$<br>$CHCOOC_2H_5$ | $CDCl_3$ δ8.03(d)1H; 7.71(s) 2H; δ7.03(dd)1H; δ6.79(d) 1H; δ5.53(q)1H; δ4.18(q) 2H; δ2.0(d)3H; δ1.17(t)3H. |
| 73 | Cl | $CF_3$ | F | $CH_3$<br>$CH(CH_2)_2COOC_2H_5$ | $CDCl_3$ δ8.01(d)1H; 7.62(s) 1H; 7.45(dd)1H; 7.07(dd)1H; 6.91(d)1H; 4.80-4.92(m)1H; 4.05(q)2H; 2.01-2.54(m)4H; 1.71(d)3H; 1.19(t)3H. |
| 74 | Cl | $CF_3$ | F | $CH_3$ O<br>CH - CH | $CDCl_3$ δ9.81(s)1H; 8.09(d) 1H; 7.64(s)1H; 7.46(dd)1H; 7.11(dd)1H; 6.78(d)1H; 5.32 (q)1H; 1.88(d)3H. |

TABLE III (Contd.)

| Compound No | $R^a$ | $R^b$ | $R^c$ | $R^4$ | Characterising Data (NMR unless otherwise stated) |
| --- | --- | --- | --- | --- | --- |
| 77 | H | $CF_3$ | CN | $CH_3$<br>$CHCOOC_2H_5$ | $CDCl_3$ δ7.94(d)1H; δ7.88(d) 1H; δ7.74(dd)1H; δ7.67(d) 1H; δ7.35(dd)1H; δ6.95(d) 1H; δ5.75(q)1H; δ4.25(q)2H; δ2.07(d)3H; δ1.25(t)3H. |

Further particular examples include Compound No. 78

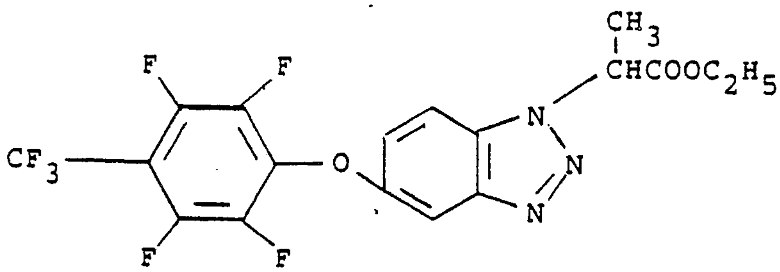

Characterising data:-
NMR: ($CDCl_3$) δ7.57(d)1H; δ7.5(d)1H; δ7.37(dd)1H; δ5.7(q)1H; δ4.21(q)2H; δ2.0(d)3H; δ1.23(t)3H.
Compound No. 79

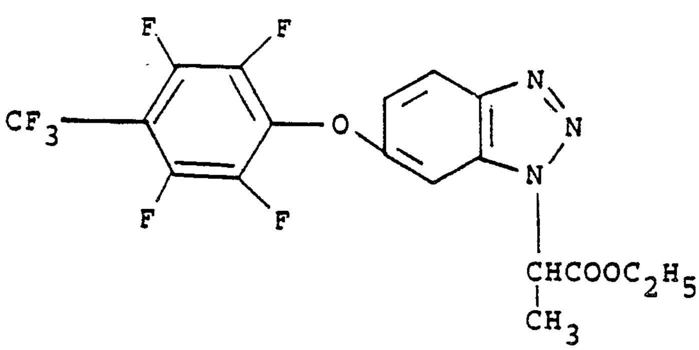

Characterising data:-
NMR: ($CDCl_3$) δ8.06(dd)1H; δ7.14(m)2H; δ5.60(q)1H; δ4.20(q)2H; δ2.0(d)3H; δ1.2(t)3H.
Compound No. 80

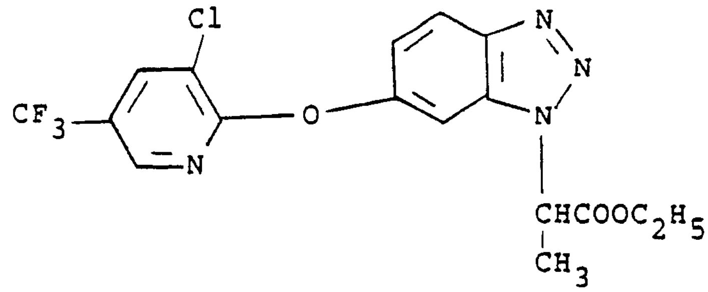

Characterising data:-

NMR: ($CDCl_3$) δ8.37(d)1H; δ8.12(d)1H; δ8.02(d)1H; δ7.4(d)1H: δ7.2(dd)1H; δ5.68(q)1H; δ4.22(q)2H; δ2.0(d)3H; δ1.22(t)3H.

Compound No. 81

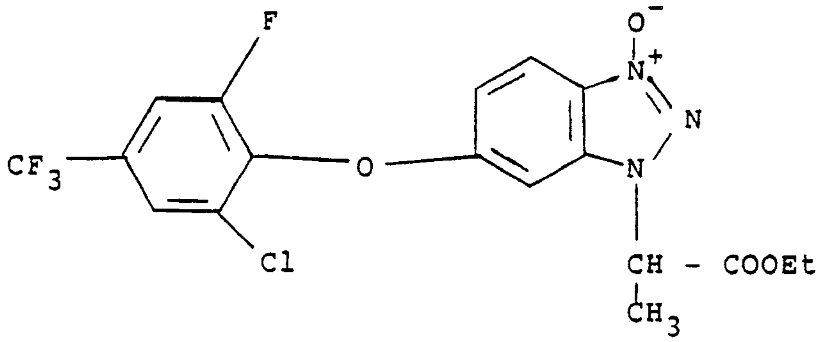

Characterising data:-

NMR: ($CDCl_3$): δ 7.97(d)1H; 7.64(s)1H; 7.06(dd) 1H; 6.78(s)1H; 5.15(q)1H; 4.17(q)2H; 1.92(d) 3H; 1.17(t)3H.

Compound No. 82

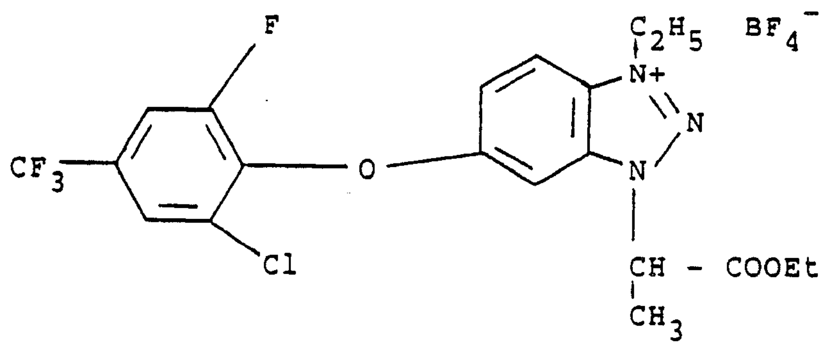

Characterising data:- M.P. 130.1 - 131.6° C

$M^+$ 460

NMR: ($CDCl_3$): δ 8.2(d)1H; 7.65(s)1H; 7.6(dd)1H; 7.49(dd)1H; 7.18(d)1H; 5.85(q)1H; 4.99(q)2H; 4.24(q)2H, 2.1(d)3H, 1.75(t)3H, 1.24(t)3H.

Compound No. 83

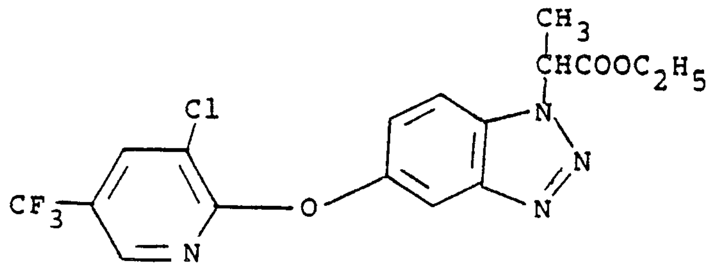

Characterising data:-
NMR: ($CDCl_3$) δ8.25(d)1H; δ8.02(d)1H; δ7.89(d)1H; 7.61(d)1H; δ7.35(dd)1H; δ5.71(q)1H; 4.23(q)2H; δ2.05(d)-3H; δ1.24(t)3H.
Compound No. 84

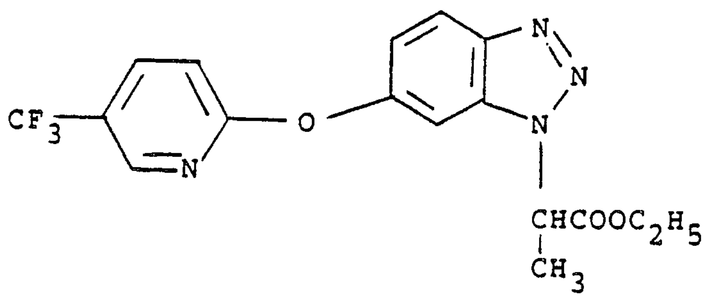

Characterising data:-
NMR: ($CDCl_3$) δ8.40(d)1H; δ8.12(d)1H; δ7.95(dd)1H; δ7.37(d)1H; δ7.18(dd)1H; δ7.1(d)1H; δ5.65(q)1H; δ4.20-(q)2H; δ2.03(d)3H; δ1.20(t)3H.
Compound No. 85

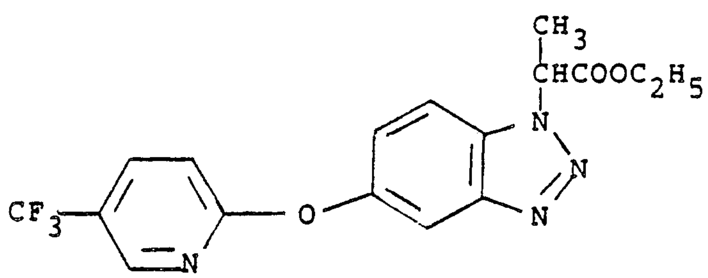

Characterising data:-
NMR: ($CDCl_3$) δ8.40(d)1H; δ7.95(dd)1H; δ7.85(d)1H; δ7.6(d)1H; δ7.33(dd)1H; δ7.1(d)1H; δ5.7(q)1H; δ4.23(q)-2H; δ2.02(d)3H; δ1.25(t)3H.
Compound No. 86

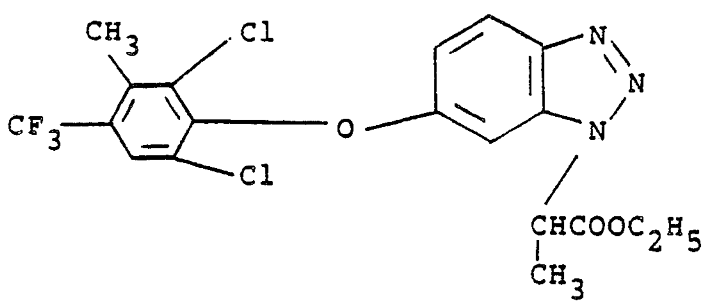

Characterising data:-
NMR: ($CDCl_3$) δ8.02(d)1H; δ7.74(s)1H; δ7.0(dd)1H; δ6.76(d)1H; δ5.52(q)1H; δ4.95(q)2H; δ2.99(q)0.2H; δ2.55-(s)2.7H; δ2.0(d)3H; δ1.26(t)0.3H; δ1.16(t)3H; and
Compound No. 87

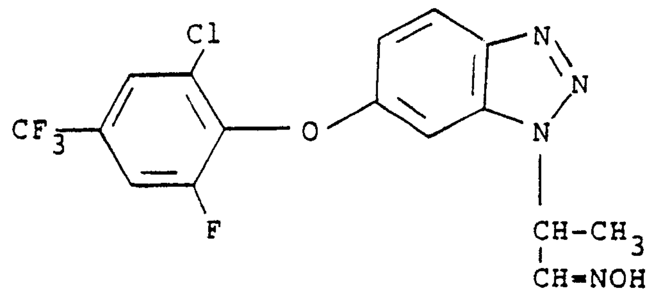

Characterising data:-
m.p. 142.8 - 145.0 °C

Compounds of formula (I) can be prepared by reacting a compound of formula (II)

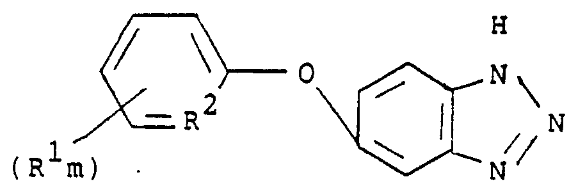

(II)

wherein $R^1$ and $R^2$ and m are as defined in relation to formula (I) with a compound of formula (III):

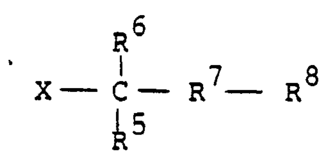

(III)

wherein $R^5$, $R^6$, $R^7$ and $R^8$ are as defined in relation to formula (I) and X is a leaving group, optionally in the presence of a base, and thereafter if desired carrying out one or more of the following steps:

i) when $R^8$ is alkoxycarbonyl hydrolysing to the corresponding acid;

ii) when $R^8$ is COOH esterifying or forming a salt, amide, sulphonamide, hydrazide or hydrazinium derivative; and

iii) when $R^8$ is an alcohol, oxidation to COOH, CHO.

Suitable leaving groups X include halogen, such as bromine and chlorine, and sulphonates such as methanesulphonate and p-toluenesulphonate.

Suitable bases for use in the reaction include alkali metal hydrides such as sodium hydride, and alkali metal carbonates and hydroxides such as potassium carbonate or sodium hydroxide.

The reaction is preferably carried out in an suitable organic solvent such as dimethylformamide, toluene, dimethylsulphoxide, a lower alkanol such as methanol or ethanol, or a lower alkyl ketone such as acetone. Moderate temperatures, for example of from 15° to 80° C are suitably employed. Conveniently the reaction is carried out at ambient temperature.

This reaction will produce a product consisting of a mixture of compounds of formula (I) wherein $R^3$ is each of the sub-groups (a), (b) and (c). These three products can be separated by conventional techniques such as chromatography in particular flash chromatography.

One compound of formula (II), is a known compound (see EP-A-0178708).

However compounds of formula (II) other than the compound 5-[2-chloro-4-(trifluoromethyl)phenoxy]-1H-benzotriazole are novel and these form a further aspect of the invention. Processes for the preparation of these compounds are described hereinafter in Schemes A, B and C.

Compounds of formula (III) are known compounds or they can be prepared from known compounds by

known methods.

Compounds of formula (I) can also be prepared by reacting a compound of formula (II) with a compound of formula (IV)

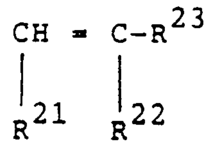

(IV)

wherein $R^{21}$ and $R^{22}$ are independently H or lower alkyl and $R^{23}$ is CN, CHO or $COOR^{24}$ where $R^{24}$ is lower alkyl optionally in the presence of a base and thereafter if desired carrying out one or more of the following steps:

i) when $R^{23}$ is alkoxycarbonyl or CN hydrolysing to the corresponding acid;

ii) when $R^{23}$ is CHO, oxidation to the corresponding acid; and

iii) from the acid formed in (i) or (ii) forming a salt, amide, ester, sulphonamide, hydrazide or hydrazanium derivative.

This Michael-type addition reaction produces compounds of formula (I) wherein $R^4$ is

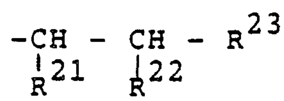

where $R^{21}$, $R^{22}$ and $R^{23}$ are as hereinbefore defined. The reaction is carried out in the presence of a base such as Triton B, alkoxides, pyridine at 40 to 100 °C as described by Wiley and Smith (JACS (1954), 76, 4933). Compounds (IV) are known compounds or they can be prepared from known compounds by known methods. This reaction also produces a mixture of compounds of formula (I) wherein $R^3$ is each of the subgroups (a), (b) and (c). These three products can be separated by conventional techniques such as chromatography and in particular flash chromatography.

In another embodiment compounds of formula (I) wherein $R^3$ is represented by the subgroup (b) can be prepared by cyclisation of compounds of formula (V)

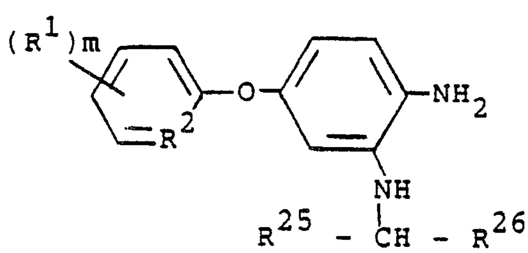

(V)

where $R^{25}$ is $CH_2OH$, COOH, $COO^-M^+$, $COOR^{29}$ or

O

P $(OC_2H_5)_2$ where $M^+$ is a cation for example sodium or potassium, $R^{29}$ is lower alkyl and $R^{26}$ is H or lower alkyl and thereafter if desired carrying out one or more of the following steps:

i) when $R^{25}$ is alkoxycarbonyl hydrolysing to the corresponding acid;

ii) when $R^{25}$ is COOH esterifying or forming a salt, amide, sulphonamide, hydrazide or hydrazinium derivative; and

iii) when $R^{25}$ is an alcohol, oxidation to COOH, CHO.

This reaction produces compounds of formula (I) wherein $R^4$ is

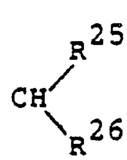

where $R^{25}$ and $R^{26}$ are as hereinbefore defined.

The cyclisation of a compound of formula (V) is carried out using a nitrite ion, provided for example by a nitrite salt such as sodium nitrite, in the presence of an acid such as a mineral acid, for example hydrochloric acid.

The reaction is suitably carried out in an aqueous solution at reduced temperature, for example of from -10 to +10 °C. Conveniently the reaction may be carried out at 0 °C to 5 °C and then allowed to warm to ambient termperature.

Compounds of formula (V) are novel and these form a further aspect of the invention. Processes for the preparation of these compounds are given hereafter (see Scheme D).

If chiral forms of compounds of formula (I) are desired, these can be produced by using chiral forms of compounds of formula (XIX) as defined hereinafter in Scheme D as starting materials for the preparation of compounds of formula (V) as described in Scheme D.

Further compounds of formula (I) may be produced by standard manipulation of the pendent $R^{26}$-CH-$R^{25}$ group. For example when $R^{25}$ is $CH_2OH$, such manipulation includes oxidation to the aldehyde group $R^{26}$-CH-CHO, acid group $R^{26}$-CH-COOH and then formation of esters, amides, salts and other derivatives or treatment with 1,1-dimethyl hydrazine to give the hydrazide $R^{25}$-CH-CONHN$(CH_3)_2$ which be further derivatised e.g. quaternised with methyl iodide to give

$R^{26}$-CH-CONH $\overset{+}{N}(CH_3)_3\ \overset{-}{I}$ .

The esters produced may be further modified by standard techniques to give the corresponding acid which may be further modified to give esters, hydrazides, hydraziniums, sulphonamides and other well known acid derivatives.

An alternative method for preparing compounds of formula (I) wherein $R^3$ is represented by the subgroup (b) and $R^4$ is represented by the group

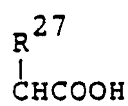

is by reacting a compound of formula (VI):

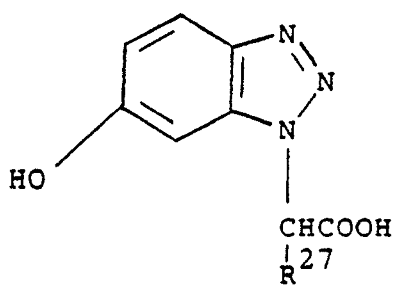

(VI)

where $R^{27}$ is H or lower alkyl with a compound of formula (VII):

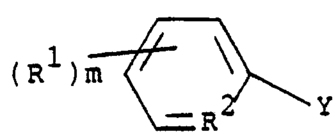

(VII)

where $R^1$, $R^2$ and m are as defined for formula (I) and Y is a halo group in the presence of a base and thereafter if desired converting the COOH group to an ester, amide, salt, sulphonamide, hydrazide or hydrazanium derivative.

The reaction is carried out in a solvent such as DMF, DMSO, DMA and lower alkyl ketones such as acetone in the presence of a base such as potassium carbonate or sodium hydroxide.

The reaction is carried out at temperatures of 25 to 120 °C, preferably 100-120 °C.

Compounds of formula (VII) are known compounds or they can be prepared from known compounds by known methods.

A further method of preparing compounds of formula (I) wherein $R^3$ is represented by subgroups (a) and (b) is by reacting a compound of formula (VIII):

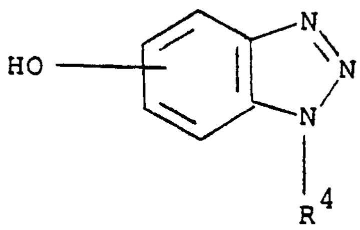

(VIII)

wherein R', m, $R^2$ and $R^4$ are as defined in formula (I) with a compound of formula (VII) as hereinbefore defined.

The reaction is carried out in a solvent such as DMF, DMSO, DMA and lower alkyl ketones e.g. acetone in the presence of a base such as potassium carbonate or sodium hydroxide.

The reaction is carried out at temperatures of 25 to 120 °C, preferably 100-120 °C.

Certain compounds of formula (VIII) are novel and these form a further aspect of the invention. In particular the invention provides compounds of formula (VIIIA).

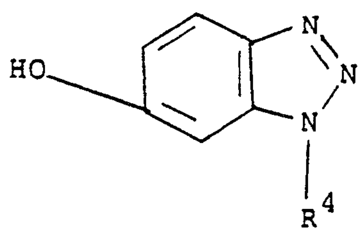

(VIIIA)

wherein $R^4$ is as hereinbefore defined.

The compounds of formula (VIII) are prepared for example as set out in Scheme E hereinafter.

Compounds of formula (II) can be prepared as set out in Scheme A.

Scheme A

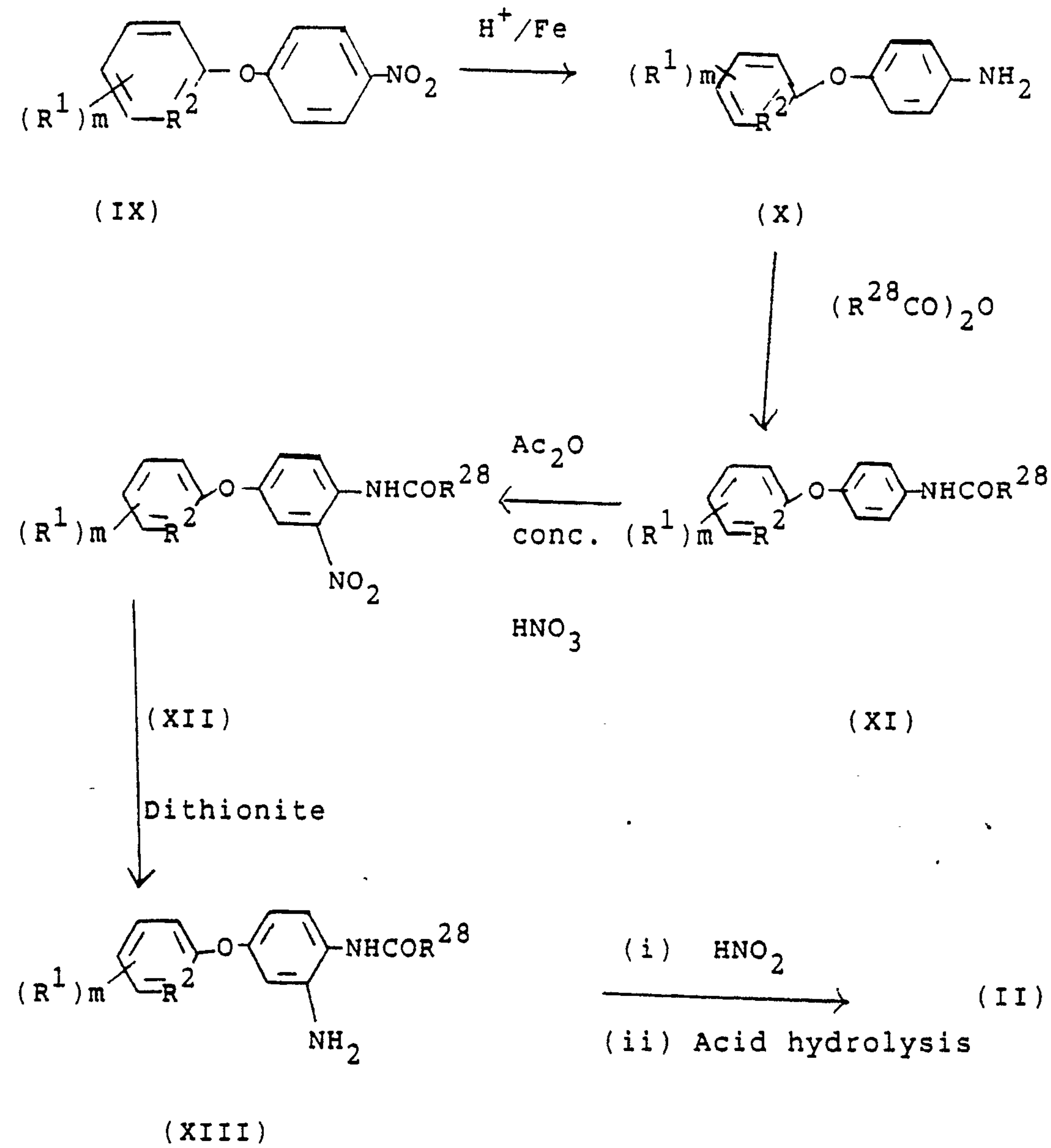


wherein $R^1$, $R^2$ and m are as defined hereinbefore; and $R^{28}$ is a $C_{1-10}$ alkyl group.

As set out in this Scheme, the compounds of formula (II) are prepared by cyclisation of a compound of formula (XIII) using a nitrite ion, provided for example by a nitrite salt such as sodium nitrite, in the presence of an acid such as a mineral acid, for example hydrochloric acid.

The reaction is suitably carried out in an aqueous solution at reduced temperature, for example of from -10 to +10°C. Conveniently the reaction may be carried out at -3 to 0°C and then allowed to warm to ambient temperature.

Compounds of formula (XIII) are prepared by reduction of the corresponding nitro compound of formula (XII). A wide variety of reducing agents may be used and may be selected from the chemical literature by the skilled worker in the art. The reduction may be carried out for example by using sodium dithionite or tin and hydrochloric acid, iron and hydrochloric acid, or hydrogen with a palladium on charcoal catalyst. The reaction is preferably effected in an organic solvent such as a lower alcohol optionally mixed with water at temperatures of from 20°C to 90°C.

Compounds of formula (XII) can be prepared by nitration of a compound of formula (XI) using a nitration agent such as concentrated nitric acid mixed with concentrated sulphuric acid or acetic anhydride. The reaction is preferably carried out in a suitable solvent such as acetic anhydride, ethylene dichloride or

concentrated sulphuric acid. Moderate temperatures of from 0° C to 25° C are suitably employed.

Compounds of formula (XI) can be prepared by reacting an amine of formula (X) with an appropriate anhydride in the presence of a base.

Suitable anhydrides include acetic anhydride (where $R^{28}$ is methyl).

Suitable bases are bases such as tertiary amines for example, triethylamine, pyridine, and 4-dimethylaminopyridine.

The reaction is suitably carried out in an inert organic solvent. A wide variety of solvents may be used, including for example lower ketones such as acetone. The reaction may be carried out for example at temperatures of from 20° C to 60° C.

The amine of formula (X) can be prepared by reduction of the corresponding nitro compound of formula (IX). Reduction is suitably effected using a reducing agent such as iron or tin and hydrochloric acid. Temperatures of from 20° C to 90° C are suitably employed. The reaction can be effected in an organic solvent such as a lower alkanol, e.g. isopropanol optionally mixed with water.

Compounds of formula (IX) are known compounds or they can be prepared from known compounds by known methods.

A further method for preparing compounds of formula (II) is set out in Scheme (B).

Scheme B

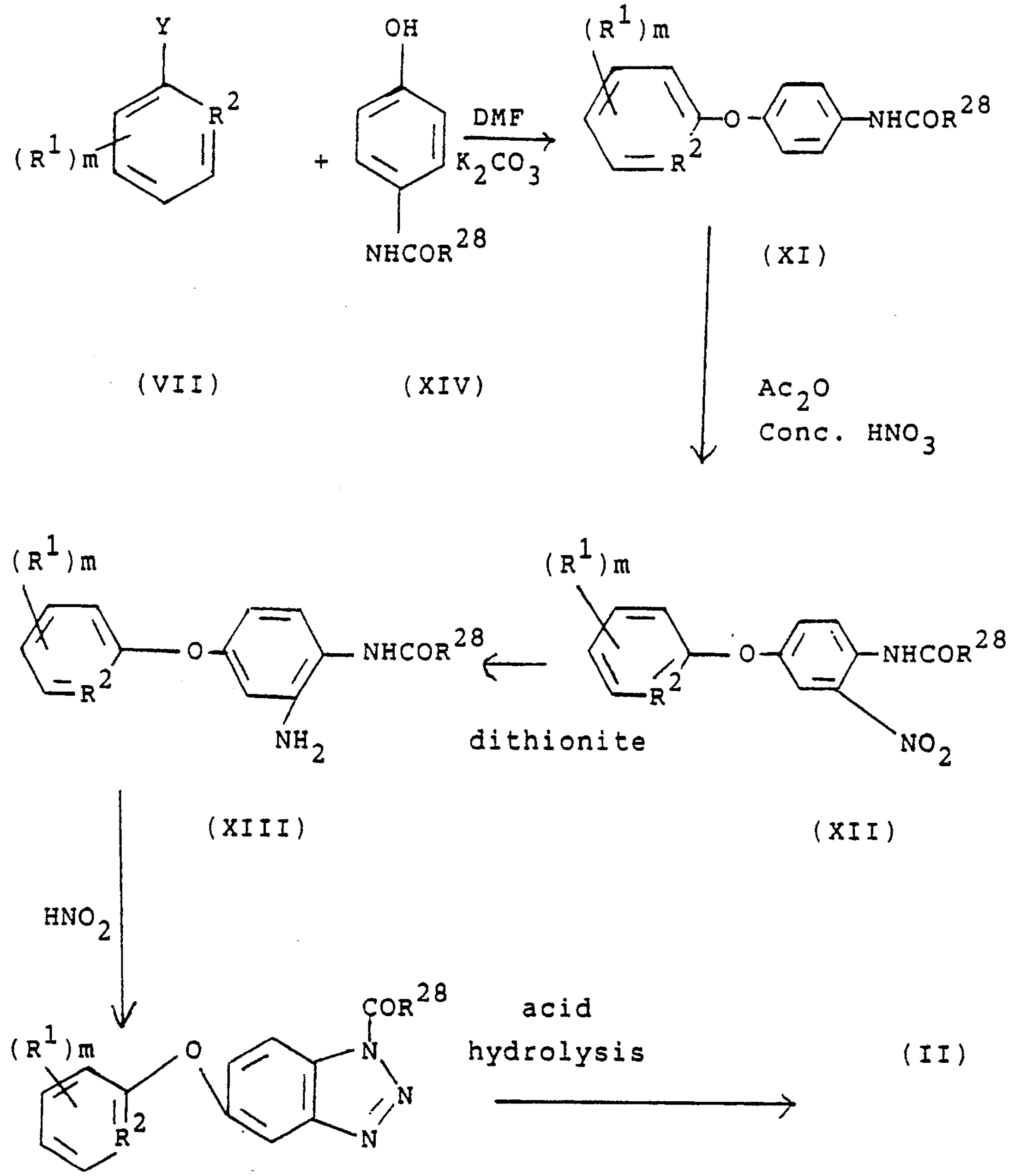


(VII) (XIV) (XI) (XII) (XIII) (II)

(XV)

wherein $R^1$, $R^2$, $R^{28}$ and m are as defined hereinbefore; and Y is a halo group.

As set out in this scheme, the compounds of formula (II) are prepared by acid hydrolysis of a compound of formula (XV) for example using concentrated hydrochloric acid and ethanol. The reaction is suitably carried out at temperatures from 20 to 80 °C.

Compounds of formula (XV) are prepared by cyclisation of compound (XIII) using a nitrite ion, provided for example by a nitrite salt such as sodium nitrite, in the presence of an acid such as a mineral acid, for example hydrochloric acid.

The reaction is suitably carried out in an aqueous solution at reduced temperature, for example of from -10 to +10 °C. Conveniently the reaction may be carried out at -3 °C to 0 °C and then allowed to warm to ambient temperature.

Compounds of formula (XIII) are prepared from compounds of formula (XII) which are prepared from compounds of formula (XI). The preparations of compounds of formulae (XIII), (XII) and (XI) are described hereinbefore in relation to Scheme A.

Compounds of formula (XI) may also be prepared as shown in this Scheme by the reaction of a compound of formula (VII) with a compound of formula (XIV) in a solvent such as DMF, DMSO, DMA and lower alkyl ketones such as acetone in the presence of a base such as potassium carbonate or sodium hydroxide.

The reaction is carried out at temperatures of 25 to 120° C, preferably 100-120° C.

Compounds of formulae (VII) and (XIV) are known compounds or they can be prepared from known compounds by known methods.

A third route to compounds of formula (II) is set out in Scheme C.

Scheme C

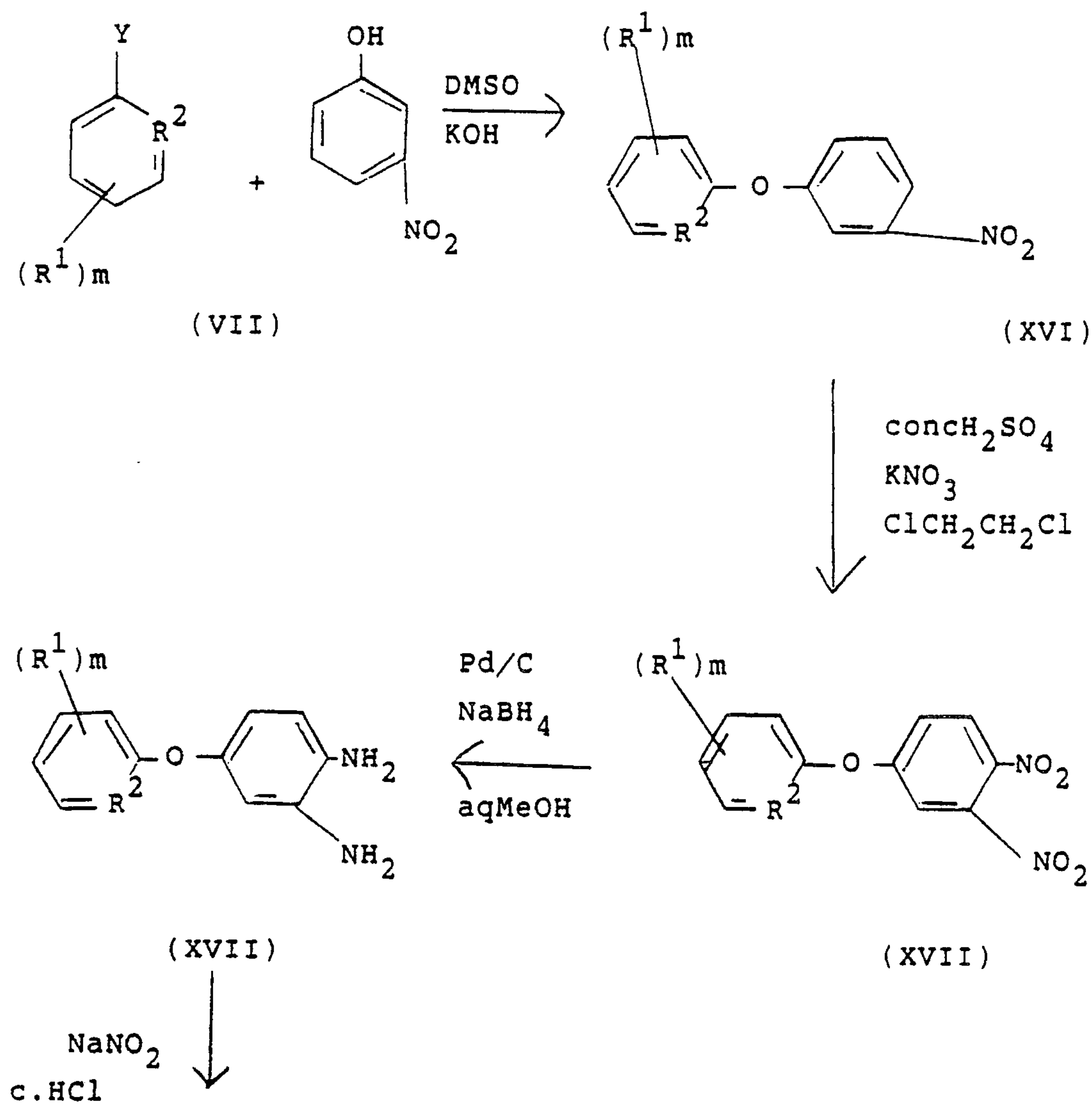


(VII)

(XVI)

(XVII)

(XVII)

(II)

A set out in this Scheme, the compounds of formula (II) are prepared by cyclisation of a compound of formula (XVIII) using a nitrite ion, provided for example by a nitrite salt such as sodium nitrite, in the presence of an acid such as mineral acid, for example hydrochloric acid.

The reaction is suitably carried out in an aqueous solution at at reduced temperature, for example of

from -10 to +10° C. Conveniently the reaction may be carried out at 0° C to 5° C and then allowed to warm to ambient temperature.

Compounds of formula (XVIII) are prepared by reduction of the corresponding dinitro compound of formula (XVII). A wide variety of reducing agents may be used and may be selected form the chemical literature by the skilled worker in the art. The reduction may be carried out for example by using sodium borohydride with a palladium on charcoal catalyst. The reaction is preferably effected in an organic solvent such as a lower alcohol optionally mixed with water at temperatures of from -20° C to 10° C.

Compounds of formula (XVII) can be prepared by nitration of a compound of formula (XVI) using a nitrating agent such as potassium nitrate mixed with concentrated sulphuric acid. The reaction is preferably carried out in a suitable solvent such as acetic anhydride, methylene dichloride, ethylene dichloride or concentrated sulphuric acid. Temperatures of from -20° C to 25° C are suitably employed.

Compounds of formula (XVI) are prepared by reacting m-nitrophenol with a compound of formula (VII) in an organic solvent, for example dimethylsulphoxide, lower alkyl ketones such as acetone, lower glymes e.g. $MeOCH_2CH_2OMe$ in the presence of a base e.g. alkaline metal hydroxides (KOH) or carbonates ($K_2CO_3$) at a temperature of 50 to 120° C.

The compounds of formula (V) can be prepared by the method shown in Scheme D.

Scheme D

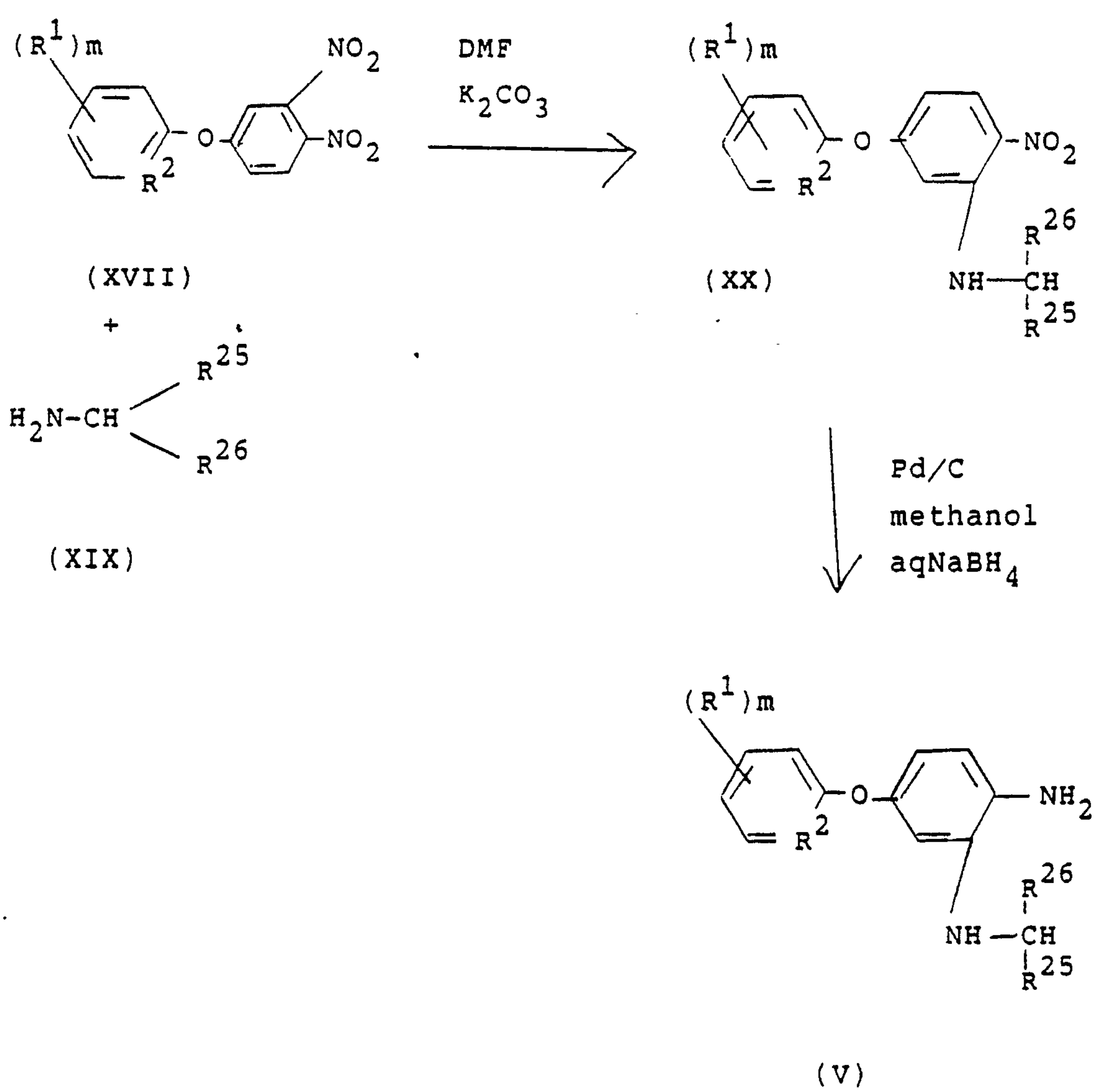


(XVII) + (XIX) → (XX) → (V)

As set out in this scheme, the compounds of formula (V) are prepared by reduction of the corresponding

nitro compound of formula (XX). Compounds of formula (XX) where $R^{25}$ is $COOR^{29}$ are first hydrolysed to the compounds of formula (XX) where $R^{25}$ is $COO^-M^+$. This hydrolysis reaction is carried out using alkaline metal hydroxides e.g. sodium or potassium hydroxide in aqueous alcoholic or THF solution at 20 °C to 50 °C. The reduction of compounds (XX) where $R^{25}$ is $COO^-M^+$ can be carried out using aqueous sodium borohydride with Pd/C at temperatures from -10 to +20 °C. When $R^{25}$ is $CH_2OH$ or

$CH_2\overset{\overset{O}{\|}}{P}(OEt)_2$ a wide variety of reducing agents may be used and may be selected from the chemical literature by the skilled worker in the art. The reduction may be carried out for example by using sodium borohydride with Pd/C or tin and hydrochloric acid, iron and hydrochloric acid, or hydrogen with a palladium on charcoal catalyst. The reaction is preferably effected in an organic solvent such as a lower alcohol optionally mixed with water at temperatures of from -5 to 90 °C.

Compounds of formula (XX) are prepared by reacting compounds of formula (XVII) with compounds of formula (XIX) in an organic solvent, for example dimethylformamide or a lower alkyl ketone or DMSO at 0 to 50 °C in the presence of a base e.g. potassium carbonate. The preparation of compounds of formula (XVII) is described in relation to Scheme C. Compounds of formula (XIX) are known compounds or they can be prepared from known compounds by known methods.

A route to compounds of formula (VIII) is set out in Scheme E.

Scheme E

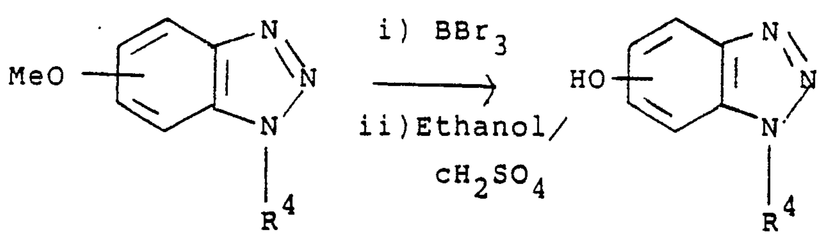


(VIII)

(XXI)

As set out in this scheme compounds of formula (VIII) are prepared by the reaction of compounds of formula (XXI) in an organic solvent for example dichloromethane using for example boron tribromide followed by ethanol/concentrated sulphuric acid. Compounds of formula (XXI) are prepared as described by Boido et al, (Studi Sassararesi, Sezione 2, 1979, 57, 787).

A route for producing compounds of formula (VI) is set out in Scheme F.

Scheme F

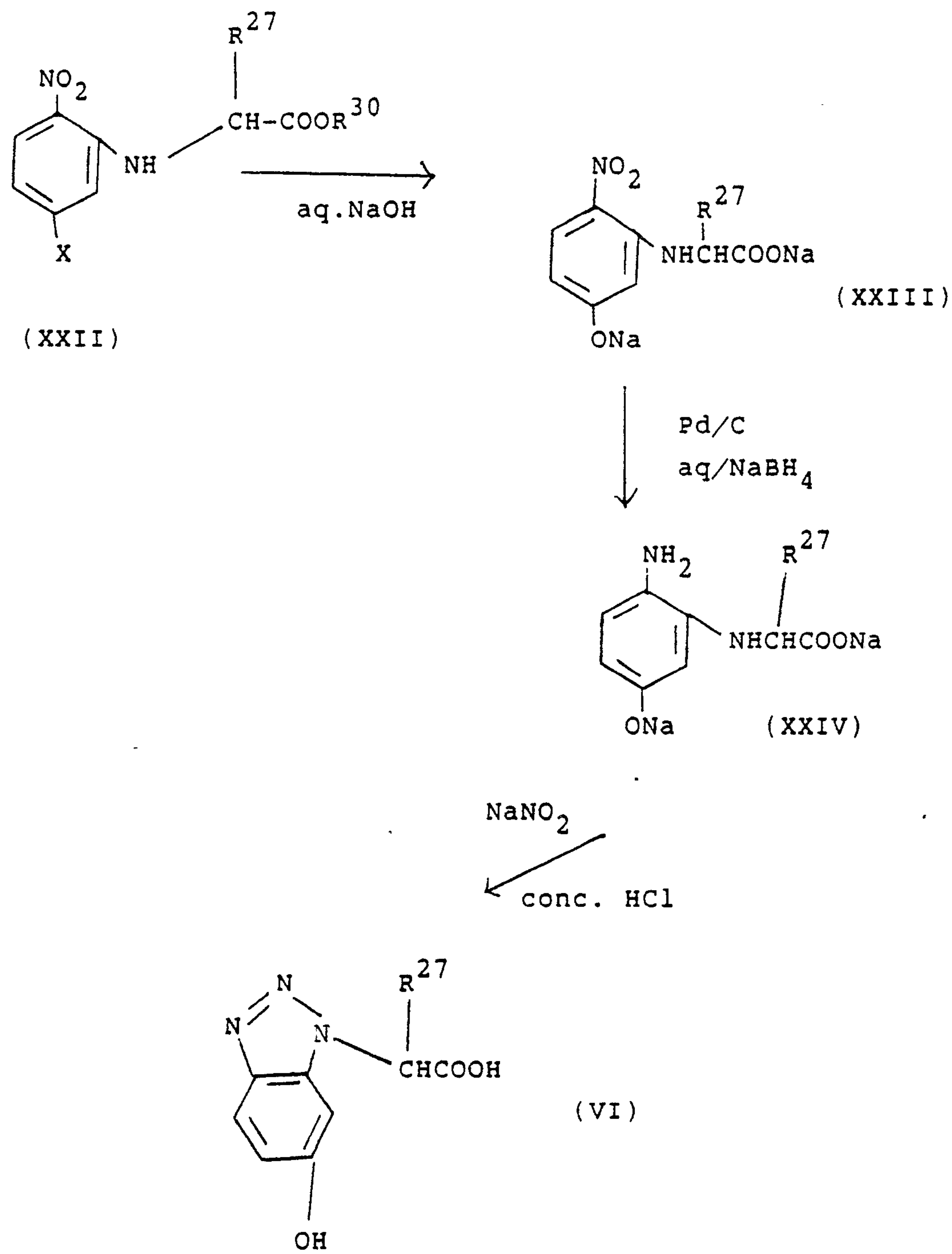


(XXII) (XXIII) (XXIV) (VI)

where $R^{27}$ is H or lower alkyl and $R^{30}$ is lower alkyl.

As set out in this scheme compounds of formula (VI) are prepared by the cyclisation of a compound of formula (XXIV) using a nitrite ion provided for example by a nitrite salt such as sodium nitrite, in the presence of an acid such as a mineral acid, for example hydrochloric acid.

The reaction is suitable carried out in an aqueous solution at reduced temperature, for example of from -10 to +10 °C. Conveniently the reaction may be carried out at 0 °C to 5 °C and then allowed to warm to ambient temperature.

Compounds of formula (XXIV) are prepared by reduction of the corresponding nitro compound of formula (XXIII). The reduction may be carried out for example by using aqueous sodium borohydride with Pd/C. The reaction is preferably effected in an organic solvent such as a lower alcohol optionally mixed with

water at temperatures of from -5 to 90° C.

Compounds of formula (XXIII) may be prepared by base hydrolysis of compounds of formula (XXII) using aqueous potassium or sodium hydroxide at temperature of 30 to 120° C.

Compounds of formula (XXII) are known compounds or they can be prepared from known compounds by known methods, as described by Crowther et al (JCS, 1949, 1260-1271), Van Dusen and Schultz (J. Org Chen., 1956, 21, 1326-29) and Fisher et al (J. Org. Chem, 1970, 35(7), 2240-2242).

The acids produced by the process may be modified if desired by esterifying or forming a salt, amide, sulphonamide, hydrazide or hydrazanium derivative.

The compounds of formula (I) are active as herbicides and therefore, in a further aspect the invention provides a process for severely damaging or killing unwanted plants which process comprises applying to the plants, or to the growth medium of the plants, an effective amount of a compound of formula (I) as hereinbefore defined.

The compounds of formula (I) are active against a broad range of weed species including monocotyledenous and dicotyledonous species. Some may show some selectivity towards certain species; they may be used as selective herbicides in rice and wheat crops.

The compounds of formula (I) may be applied directly to the plant (post-emergence application) or to the soil before the emergence of the plant (pre-emergence application). They are particularly useful when applied post-emergence.

The compounds of formula (I) may be used on their own to inhibit the growth of, severely damage, or kill plants but are preferably used in the form of a composition comprising a compound of the invention in admixture with a carrier comprising a solid or liquid diluent.

Therefore, in yet a further aspect the invention provides plant growth inhibiting, plant damaging, or plant killing compositions comprising a compound of formula (I) as hereinbefore defined and an inert carrier or diluent.

Compositions containing compounds of formula (I) include both dilute compositions, which are ready for immediate use, and concentrated compositions, whlich require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, e.g. kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type or mixtures thereof. The cationic agents are, for example, quaternary ammonium compounds (e.g. cetyltrilmethylammonium bromide). Suitable anionic agents are soaps; salts of aliphatic mono ester of sulphuric acid, for example sodium lauryl sulphate; and salts of sulphonated aromatic compounds, for example sodium dodecylbenzenesulphonate, sodium,calcium, and ammonium lignosulphonate, butylnaphthalene sulphonate, and a mixture of the sodium salts of diisopropyl and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol (e.g. Agral 90) or octyl-cresol. Other non-ionic agents are the partial esters derived from long chain fatty acids and hexitol anhydrides, for example sorbitan monolaurate; the condensation products of the partial ester with ethylene oxide; and the lecithins; silicone surface active agents (water soluble surface active agents having a skeleton which comprises a siloxane chain e.g. Silwet L77). A suitable mixture in mineral oil is Atplus 411F.

The aqueous solutions or dispersions may be prepared by dissolving the active ingredient in water or an organic solvent optionally containing wetting or dispersing agent(s) and then, when organic solvents are used, adding the mixture so obtained to water optionally containing wetting or dispersing agent(s). Suitable organic solvents include, for example, ethylene di-chloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then diluted with water before use. The concentrates are usually required to withstand storage for prolonged

periods and after such storage, to be capable of dilution with water to form aqueous preparations which remain homogeneous for a sufficient time to enable them to be applied by conventional spray equipment. Concentrates conveniently contain 20-90%, preferably 20-70%, by weight of the active ingredient(s). Dilute preparations ready for use may contain varying amounts of the active ingredient(s) depending upon the intended purpose: amounts of 0.01% to 10.0% and preferably 00.1% to 2%, by weight of active ingredient-(s) are normally used.

A preferred form of concentrated composition comprising the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties too, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited, the formulations selected for use and whether the compound is to be applied for foliage or root uptake. As a general guide, however, an application rate of from 0.001 to 20 kilograms per hectare is suitable while from 0.025 to 10 kilograms per hectare may be preferred.

The compositions of the invention may comprise, in addition to one or more compounds of the invention, one or more compounds not of the invention but which possess biological activity. Accordingly in yet a still further embodiment the invention provides a herbicidal composition comprising a mixture of at least one herbicidal compound of formula (I) as hereinbefore defined with at least one other herbicide.

The other herbicide may be any herbicide not having the formula (I). It will generally be a herbicide having a complementary action in the particular application.

Examples of useful complementary herbicides include:

A. benzo-2,1,3-thiodiazin-4-one-2,2-dioxides such as 3-isopropylbenzo-2,1,3-thiadiazin-4-one-2, 2-dioxide (bentazone);

B. hormone herbicides, particularly the phenoxy alkanoic acids such as 4-chloro-2-methylphenoxy acetic acid (MCPA), S-ethyl 4-chloro-O-tolyloxy thio-acetate (MCPA-thioethyl) 2-(2,4- dichlorophenoxy) propionic acid (dichlorprop), 2,4,5-trichlorophenoxyacetic acid (2,4,5-T), 4-(4-chloro-2-methylphenoxy)butyric acid (MCPB), 2,4-dichlorophenoxyacetic acid (2,4-D), 4-(2,4-dichlorophenoxy)butyric acid (2,4-DB), 2-(4-chloro-2-methylphenoxy) propionic acid (mecoprop), 3,5,6-trichloro-2-pyridyloxyacetic acid (trichlopyr), 4-amino-3,5-dichloro-6- fluoro-2-pyridyloxyacetic acid (fluroxypyr), 3,6-dichloropyridine-2-carboxylic acid (clopyralid), and their derivatives (eg. salts, esters and amides);

C. 1,3-dimethylpyrazole derivatives such as 2-[4-(2,4-dichlorobenzoyl) 1,3-dimethylpyrazol-5- yloxy]-acetophenone (pyrazoxyfen), 4-(2,4-dichlorobenzoyl)1,3-dimethylpyrazol-5-yltoluene suphonate (pyazolate) and 2-[4-(2,4-dichloro-m-toluolyl)-1,3-dimethylpyrazol-5-yloxy]-4′-methylacetophenone (benzofenap);

D. Dinitrophenols and their derivatives (eg. acetates such as 2-methyl-4,6-dinitrophenol (DNOC), 2-t-butyl-4,6-dinitrophenol (dinoterb), 2-secbutyl-4,6-dinitrophenol (dinoseb) and its ester, dinoseb acetate;

E. dinitroaniline herbicides such as N′, N′-diethyl-2,6-dinitro-4-trifluoromethyl-m-phenylenediamine (dinitramine), 2,6-dinitro-N,N-dipropyl-4-trifluoro-methylaniline (trifluralin), N-ethyl-N-(2-methylallyl)-2,6-dinitro-4-trifluoromethylaniline (ethalfluralin), N-(1-ethylpropyl)-2,6-dinitro-3,4-xylidine (pendimethalin); and 3,5-dinitro-$N^4$, $N^4$-dipropylsulphanilamide (oryzalin);

F. arylurea herbicides such as N′-(3,4-dichlorophenyl)-N,N-dimethylurea (diuron), N,N-dimethyl-N′-[3-(trifluoromethyl) phenyl]urea (flumeturon), 3-(3-chloro-4-methoxyphenyl)-1, 1-dimethylurea(metoxuron), 1-butyl-3-(3,4-dichlorophenyl)-1-methylurea(neburon), 3-(4-isopropylphenyl)-1, 1-dimethylurea (isoproturon), 3-(3-chloro-p-tolyl)-1,1-dimethylurea (chlorotoluron), 3-[4-(4- chlorophenoxy) phenyl]-1, 1-dimethylurea (chloroxuron), 3-(3,4-dichlorophenyl)-1-methylurea (linuron), 3-(4-chlorophenyl)-1-methoxy-1-methylurea (monolinuron), 3-(4-bromo-4-chlorophenyl)-1-methoxy-1-methylurea (chlorobromuron), 1-(1-methyl-1-phenylethyl)-3-p-tolylurea(daimuron), and 1-benzothiazol-2-yl-1, 3-dimethylurea (methabenzthiazuron);

G. phenylcarbamoyloxyphenylcarbamates such as 3-[methoxycarbonylamino]phenyl (3-methylphenyl)-carbamate (phenmedipham) and 3-[ethoxycarbonylamino]-phenyl phenylcarbamate (desmedipham);

H. 2-phenylpyridazin-3-ones such as 5-amino-4-chloro-2-phenylpyridazin-3-one (chloridazon), and 4-chloro-5-methylamino-2-(α, α, α-trifluoro- m-tolyl) pyridazin-3(2H)-one (norflurazon);

I. uracil herbicides such as 3-cyclohexyl-5,6-trimethyleneuracil (lenacil), 5-bromo-3-sec-butyl-6-methyl-uracil (bromacil) and 3-t-butyl-5-chloro-6-methyl-uracil (terbacil);

J. triazine herbicides such as 2-chloro-4-ethylamino-6-(i-propylamino)-1,3,5-triazine (atrazine), 2-chloro-4,6-di(ethylamino)-1,3,5-triazine (simazine), 2-azido-4-(i-propylamino)-6-methylthio-1,3,5-triazine (aziprotryne), 2-(4-chloro-6-ethylamino-1,3,5-triazin-2-ylamino)-2-methylpropionitrile (cyanazine), $N^2$,$N^4$-di-isopropyl-6-methylthio-1,3,5-triazine-2,4-diamine (prometryn), $N^2$-(1,2-dimethylpropyl)-$N^4$-ethyl-6-methylthio-1,3,5-triazine-2,4 -diamine (dimethametryn), $N^2$,$N^4$-diethyl-6- methylthio-1,3,5-triazine-2,4-diamine (simetryne), and $N^2$-tert-butyl-$N^4$-ethyl-6-methylthio-1,3,5-triazine-2,4-diamine (terbutryn);

K. phosphorothioate herbicides such as S-2-methylpiperidinocarbonyl-methyl O,O-dipropyl phosphorodithioate (piperophos), S-2-benzenesulphonamidoethyl O,O-diisopropyl phosphonodithioate (bensulide), and O-ethyl O-6-nitro-m-tolyl sec-butylphosphoamidothioate (butamifos);

L. thiolcarbamate herbicides such as S-ethyl N-cyclohexyl-N-ethyl(thiocarbamate) (cycloate), S-propyl dipropyl-thiocarbamate (vernolate), S-ethyl-azepine-1-carbothioate (molinate), S-4-chlorobenzyl diethyl-thiocarbamate (thiobencarb), S-ethyl di-isobutyl- thiocarbamate (butylate)*, S-ethyl diisopropylthiocarbamate (EPTC)*, S-2,3,3-trichloroallyl di-isopropyl (thiocarbamate) (tri-allate), S-2, 3-dichloroallyl di-isopropyl (thiocarbamate) (di-allate), S-benzyl 1,2-dimethylpropyl (ethyl) thiocarbamate (esprocarb), S-benzyl di(sec-butyl)-thiocarbamate (tiocarbazil), 6-chloro-3-phenylpyridazin 4-yl S-octyl thiocarbamate (pyridate), and S-1-methyl-1- phenylethylpiperidine-1-carbothioate (dimepiperate);
(*These compounds are preferably employed in combination with a safener such as 2,2-dichloro-N,N-di-2-propenylacetamide (dichlormid)),

M. 1,2,4-triazin-5-one herbicides such as 4-amino-4,5-dihydro-3-methyl-6-phenyl-1,2,4-triazine-5-one-(metamitron) and 4-amino-6-t-butyl-4,5-dihyrdro-3-methylthio-1,3,4-triazin-5-one (metribuzin);

N. benzoic acid herbicides such as 2,3,6-trichloro-benzoic acid (2,3,6-TBA), 3,6-dichloro-2-methoxybenzoic acid (dicamba) and 3-amino-2, 5-dichloro benzoic acid (chloramben);

O. anilide herbicides such as 2-chloro-2′,6′-diethyl-N-(2-propoxyethyl)acetanilide (pretilachlor), N-butoxymethyl-chloro-2′,6′-diethylacetanilide (butachlor), the corresponding N-methoxy compound (alachlor), the corresponding N-i-propyl compound (propachlor), 3′,4′-dichloropropionilide (propanil), 2-chloro-N-[pyrazol-1- ylmethyl]acet-2′-6′ xylidide(metazachlor), 2-chloro-6′-ethyl-N-(2-methoxy-1-methylethyl) acet-0-toluidide (metolachlor), 2-chloro-N-ethoxymethyl-6′-ethylacet-0-toluidide (acetochlor), and 2-chloro-N-(2-methoxyethyl)acet-2′,6′-xylidide (dimethachlor);

P. dihalobenzonitrile herbicides such as 2,6-dichloro-benzonitrile (dichlobenil), 3,5-dibromo-4-hydroxy-benzonitrile (bromoxynil) and 3,5-diiodo-4-hydroxy-benzonitrile (ioxynil);

Q. haloalkanoic herbicides such as 2,2-dichloropropionic acid (dalapon), trichloroacetic acid (TCA) and salts thereof;

R. diphenylether herbicides such as ethyl 2-[5-(2-chloro-trifluoro-p-tolyloxy)-2- nitrobenzoylooxy propionate (lactofen), D-[5-(2-chloro-α,α,α-trifluoro-p-tolyloxy)-2- nitrobenzoyl] glycolic acid (fluroglycofen) or salts or ester thereof, 2,4-dichlorophenyl-4- nitrophenyl ether (nitrofen), methyl-(2,4- dichlorophenoxy)-2-nitrobenzoate (bifenox), 2-nitro-5-(2-chloro-4-trifluoromethyl-phenoxy) benzoic acid (acifluorofen) and salts and esters thereof, 2-chloro-4-trifluoromethylphenyl 3-ethoxy-4-nitrophenyl ether (oxyfluorfen) and 5-(2-chloro-4-(trifluoromethyl) phenoxy)-N-(methylsulfonyl)-2-nitrobenzamide (fomesafen); 2,4,6-trichlorophenyl 4-nitrophenyl ether (chlornitrofen) and 5-(2,4-dichlorophenoxy)-2-nitroanisole (chlomethoxyfen);

S. phenoxyphenoxypropionate herbicides such as (RS)-2-[4-(2,4-dichloro-phenoxy)phenoxy) propionic acid (diclofop) and esters thereof such as the methyl ester, 2-(4-(5-trifluoromethyl)-2-(pyridinyl)oxy) phenoxypropanoic acid (fluazifop) and esters thereof, 2-(4-(3-chloro-5-trifluoro-methyl)-2-pyridinyl)oxy)phenoxy)-propanoic acid (haloxyfop) and esters thereof, 2-(4-((6-chloro-2-quinoxalinyl)oxy) phenoxypropanoic acid (quizalofop) and esters thereof and (±)-2-[4-(6-chlorobenzoxazol-2-yloxy)-phenoxy]propionic acid (fenoxaprop) and esters thereof such as the ethyl ester;

T. cyclohexanedione herbicides such as 2,2-dimethyl-4,6-dioxo-5-(1-((2-propenyloxy)-imino)-butyl) cyclohexane carboxylic acid (alloxydim) and salts thereof, 2-(1-ethoxyimino) butyl-5-(2-(ethylthio)-propyl)-3-hydroxy-2-cyclohexan-1- one(sethoxydim), 2-(1-ethoxyimino) butyl)-3 -hydroxy-5-thian-3-ylcyclohex-2-enone (cycloxydim)2-[1-(ethoxyimino)propyl]-3-hydroxy-5-mesitylcyclohex-2-enone(tralkoxydim), and (±)-2-{(E)-1-[-(E)-3-chloroallyloximino] propyl}-5-[2-(ethylthio)propyl]-3-hydroxy-cyclohex-2-enone (clethodim);

U. sulfonyl urea herbicides such as 2-chloro-N (4-methoxy-6-methyl-1,3,5-triazin-2-yl)-aminocarbonyl) benzenesulphonamide (chlorosulfuron), methyl 2-((((4,6-dimethyl-2-pyrimidinyl)amino)-carbonyl)amino)-sulphonylbenzoic acid (sulfometuron), 2-(((3-(4-methoxy-6-methyl-1,3,5-triazin-2-yl)carbonyl) amino)-sulphonyl)-benzoic acid (metsulfuron) and esters thereof;
-(4,6-dimethoxypyrimidin-2-ylcarbamoylsuphamoy)-O-tol uic acid (benzsulfuron) and esters thereof such as the methyl3-[3-(4-methoxy-6methyl-1,3,5-triazin-2-yl)ureidosulphonyl]thiophene-2-carboxylate(DPX-M6313), 2-(4-chloro-6-methoxy pyrimidin-2-yl carbamoylsulphamoyl benzoic acid (chlorimuron) and esters such as the ethyl ester thereof 2-(4,6-dimethoxypyrimidin-2-ylcarbamoylsulphamoyl)-N, N-dimethyl-nicotinamide, 2-[4,6-bis(difluoromethoxy)pyrimidin-2-ylcarbamoylsulphamoyl) benzoic acid (pirimisulfuron) and esters such

as the methyl ester thereof. 2-[3-(4-methoxy-6-methyl-1,3,5-triazin-zyl)-3-methylureidosulphonyl) benzoic acid estes such as the methyl ester thereof (DPX-LS300) and5-(4,6-dimethoxypyrimidin-2-ylcarbamoylsulphamoyl)-1-methylpyrazole-4- carboxylic acid (pyrazosulfuron);

V. imidazolidinone herbicides such as 2-(4,5-dihydro-4-isopropyl-4-methyl-5-oxoimidazol-2- yl) quinoline-3-carboxylic acid (imazaquin), methyl 6-(4-isopropyl-4-methyl-5-oxo-2-imidazolin-2-yl)-m-toluate and p-toluateisomer(imazamethabenz),2-(4-isopropyl-4-methyl- 5-oxo-2-imidazolin-2-yl) nicotinic acid (imazapyr) and isopropylammonium salts thereof, (RS)-5-ethyl-2-(4-isopropyl-4-methyl-5-oxo-2-imidazolin-2-yl)nicotinic acid (imazethapyr);

W. arylanilide herbicides such as benzoyl-N-(3-chloro-4-fluorophenyl)-L-alanine (flamprop) and esters thereof, ethyl N-benzoyl-N-(3,4-dichlorophenyl)-DL-alaninate (benzoylprop -ethyl), N-(2,4-difluorophenyl)-2-(3-trifluoromethyl)phenoxy)-3-pyridinecarboxamide (diflufenican);

X. amino acid herbicides such as N-(phosphonomethyl)-glycine (glyphosate) and DL-homoalanin-4-yl (methyl)-phosphinic acid (glufosinate) and their salts and esters, trimethylsulfonium N-(phosphonomethyl)-glycine (sulphosate), and bilanafos;

Y. organoarsenical herbicides such as monosodium methanearsonate (MSMA);

Z. herbicidal amide derivative such as (RS)-N,N-diethyl-2-(1-naphthyloxypropionamide) (napropamide), 3,5-dichloro-N-(1,1- dimethylpropynyl)benzamide (propyzamide), (R)-1-(ethylcarbamoyl)ethyl carbanilate (carbetamide), N-benzyl-N-isopropylpivalamide (tebutam), (RS)-2-bromo-N-($\alpha$,$\alpha$-dimethylbenzyl)-3,3-dimethylbutyramide (bromobutide), N-[3-(1-ethyl-1-methylpropyl)-isoxazol-5-yl] 2,6-dimethoxybenzamide, (isoxaben), N-phenyl-2-(2-naphthyloxy) propionamide (naproanilide), N,N-dimethyl-diphenylacetamide (diphenamid), and N-(1-naphthyl)-phthalamic acid (naptalam);

AA. miscellaneous herbicides including 2-ethoxy-2,3-dihydro-3, 3-dimethylbenzofuran methanesulfonate (ethofumesate), 7-oxabicyclo (2.2.1)heptane,1-methyl-4-(1-methylethyl)-2-(2-methylphenylmethoxy)-exo (cinmethylin), 1,2-dimethyl-3,5-diphenylpyrazolium ion (difenzoquat) and salts thereof such as the methyl sulphate salt, 2-(2-chlorobenzyl)-4,4- dimethyl-1,2-oxazoldin -3-one (clomazone), 5-tert-butyl-3-(2,4-dichloro-5-isopropoxyphenyl)-1,3,4-oxadiazol-2(3H)-one (oxadiazon), 3,5-dibromo-4-hydroxy benzaldehyde 2,4-dinitrophenyloxime (bromofenoxim), 4-chlorobut-2-ynyl-3-chlorocarbanilate (barban), (RS)-2-(3,5-dichlorophenyl)-2-(2,2,2-trichloroethyl)oxirane (tridiphane), (3RS, 4RS; 3RS,4SR)-3-chloro-4-chloromethyl-1-$\alpha$,$\alpha$,$\alpha$-trifluro-m-totyl)-2-pyrrolidone (in the ratio 3:1) (flurochloridone), dichloroquinoline 8-carboxylic acid (quinchlorac) and 2-(1,3- benzothiazol-2-yl-oxy)-N-methylacetanilide (mefanacet);

BB. Examples of useful contact herbicides include:

bipyridylium herbicides such as thos in which the active entity is the 1,1′-dimethyl-4,4′ -dipyridylium ion (paraquat) and those in which the active entity is the 1,1′-ethylene-2,2′ -dipyridylium ion (diquat);

The following Examples illustrate the invention.

## EXAMPLE 1

This Example illustrates the preparation of compound 1 in Table I, compound 2 in Table II and compound 3 in Table III.

Step A

4-(2′,4′-dichlorophenoxy)nitrobenzene (1.7g) was dissolved in isopropanol (28 $cm^3$) and reduced iron powder (3.73g) was added followed by water (6 $cm^3$) and concentrated hydrochloric acid (0.23 $cm^3$). The reaction mixture was stirred under reflux for 45 minutes and then filtered through Hyflo while hot. The solvent was removed from the filtrate under reduced pressure. The residue was partitioned between ethyl acetate/water. The organic extract was washed with three further portions of water, then dried over $MgSO_4$. The extract was filtered and the solvent removed from the filtrate under reduced pressure. The residue, a dark brown oil, was purified by flash chromatography using $SiO_2$ $CHCl_3$: $Et_2O$, 95:5 as eluent of 4-(2′,4′-dichlorophenoxy)benzamine (1.18g) was obtained as a straw coloured oil (75.4%).

Step B

The benzamine from Step (A) (31.8g) was dissolved in acetone (134 $cm^3$) and triethylamine (27 $cm^3$)

was added followed by acetic anhydride (20 $cm^3$)with stirring. The reaction mixture was stirred under reflux for $1\frac{1}{2}$ hours. The solvent was removed under reduced pressure and the residue was partitioned between ethyl acetate/water. The aqueous phase was discarded and the organic extract was washed once more with water, once with saturated $NaHCO_3$ solution and then three times with water. The organic extract was dried over $MgSO_4$, filtered and the solvent removed from the filtrate under reduced pressure. The residue was recrystallised from ethanol and air dried to give 4-(2′,4′-dichlorophenoxy)acetanilide (31.4g); m.p. 143.7-144.6° C).

Step C

The acetanilide from Step (B) (23.3g) was suspended in acetic anhydride (83 $cm^3$) acid and cooled in a salt ice bath to -8° C. Concentrated nitric acid (4.96 $cm^3$) was dissolved in acetic anhydride (9.92 $cm^3$) at -5° C, and this solution was added dropwise with stirring and cooling to the reaction mixture, so that the final temperature was -5° C.

The reaction mixture was allowed to warm to room temperature with stirring, and then allowed to stand at room temperature for 1 hour. Water (200 $cm^3$) was added to the reaction mixture with vigorous stirring. The bright yellow product was filtered off under reduced pressure and washed with three further portions of water. The product was air dried then recrystallised from ethanol. The product was air dried to give 4-(2′,4′-dichlorophenoxy)-2-nitroacetanilide (21.23g); m.p. 129-133.3° C.

Step D

The nitroacetanilide from Step (C) (21.23g) was dissolved in hot ethanol (750 $cm^3$) and sodium dithionite (43.58g) was added portionwise (10 portions) over $1\frac{1}{2}$ hours, each portion being followed by water (20 $cm^3$). After the addition, the reaction mixture was stirred under reflux for $1\frac{1}{2}$ hours then left to stand overnight at room temperature. The solvent was removed under reduced pressure and more ethanol was added and removed also under reduced pressure. Ethyl acetate was added and the mixture stirred at 40° C until all lumps had been broken up. The mixture was filtered to remove the inorganics and the solvent removed from the filtrate under reduced pressure. 4-(2′,4′-dichlorophenoxy)-2-aminoacetanilide was obtained as a sticky foam which became brittle on cooling.

Step E

The amine from step (D) (5g) was stirred with water (61 $cm^3$) and concentrated hydrochloric acid (7.4 $cm^3$) was added. The reaction was stirred at room temperature for 15 minutes after which more water (54 $cm^3$) was added and the reaction cooled in a salt/ice bath to -3° C. A solution of sodium nitrite (2.47g) in water (25 $cm^3$) was added slowly with constant stirring and cooling to -3° C. After the addition, the reaction mixture was stirred below 0° C for 30 minutes, allowed to warm to room temperature and then stirred for 3 hours. The reaction mixture was extracted three times with ethyl acetate. The organic extracts were bulked and washed three times with water. The organic extract was dried over $MgSO_4$, filtered, and the solvent removed from the filtrate under reduced pressure. The residue was a dark brown oil, which was purified by flash chromatography using $SiO_2$, trichloromethane as eluent to give 1-acetyl-5-(2′,4′-dichlorophenoxy)-benzotriazole, as a straw coloured oil (yield = 2.35g).

Step F

The amide from Step (E) (1.85g) was dissolved in ethanol (5 $cm^3$) and concentrated hydrochloric acid (3 $cm^3$) and stirred under reflux for $2\frac{1}{2}$ hours. The reaction mixture was left to stand at room temperature overnight. The reaction was partitioned between water/ethyl acetate and the organic extract separated. The aqueous phase was extracted with three further portions of ethyl acetate. The organic extracts were bulked and washed four times with water. The organic extract was dried over $MgSO_4$, filtered and the solvent removed from the filtrate under reduced pressure to give 5-(2′,4′-dichlorophenoxy)-benzotriazole, (1.65g).

Step G

The benzotriazole from Step (F) (1.62g) was dissolved in dry dimethylformamide (20 $cm^3$) and 50% NaH (0.28g) added slowly with stirring. The reaction mixture was stirred at room temperature for 20 minutes and then ethyl-2-bromopropionate (1.04g) added. The reaction mixture was stirred at room temperature for 16 hours. The reaction mixture was diluted with water (200 $cm^3$) and extracted four times with ethyl acetate. The organic extracts were bulked and washed with water. The organic extract was dried over $MgSO_4$, filtered, and the solvent removed from the filtrate under reduced pressure. The residue was a straw coloured oil. The oil was purified by flash chromatography on $SiO_2$ using trichloromethane:diethylether, 96:4 as eluent. Three colourless oils were obtained which were shown by n.m.r. to be compound 1 Table I, compound 2 in Table II and compound 3 in Table III.

Compounds 4, 5, 6, 31, 32, 33, 34, 35, 36 43, 47, 48, 52 and 53 were prepared by analogous methods using appropriate reactants.

EXAMPLE 2

This Example illustrates the preparation of compound 10 in Table I, compound 11 in Table II and compound 12 in Table III.

Step A

p-acetamidophenol (1g) and 5-chloro-3,4-difluorobenzotrifluoride (1.43g) were dissolved in dry DMF (4 $cm^3$). Dry potassium carbonate (1.37g) was added and the mixture stirred and heated at 110 to 120°C for 2 hours. The reaction mixture was cooled, poured into water (40 $cm^3$) and extracted three times with ethyl acetate. The combined organic extracts were washed three times with water, dried ($MgSO_4$) filtered and the solvent removed from the filtrate under vacuum. The residue was recrystallised from toluene to give 4-(2′-chloro-4′-trifluoromethyl-6′-fluorophenoxy) acetanilide, (135g, 59%) as a white solid.

Step B

A cold solution of concentrated nitric acid (0.2 $cm^3$) in acetic anhydride (1 $cm^3$) was added dropwise to a stirred, cooled (-5°C) suspension of 4-(2′-chloro-4′-trifluoromethyl-6′-flurophenoxy) acetanilide (1.12g) in acetic anhydride (4 $cm^3$). After 10 minutes at -5°C, the mixture was stirred at room temperature for 5 hours. The mixture was diluted with water and the solid removed by filtration, washed with water and air dried. Recrystallisation from toluene gave 4-(2′-chloro-4′-trifluoromethyl-6′-fluorophenoxy)-2-nitroacetanilide (0.54g, 43%), as a yellow solid, m.p. 162.5-163°C.

Step C

4-(2′-chloro-4′-trifluoromethyl-6′-fluorophenoxy)-2-nitroacetanilide (35.35g) was dissolved in stirred, hot ethanol (1L) and sodium dithionite (63.1g) added in 10 portions over $1\frac{1}{2}$ hours, each portion being followed by water (29 $cm^3$). When addition was complete, the mixture was stirred under reflux for 1.5 hours. The solvent was removed from the mixture under vacuum and the residue extracted with ethyl acetate. Removal of the ethyl acetate after filtration gave crude 4-(2′-chloro-4′-trifluoromethyl-6′-fluorophenoxy)-2-aminoacetanilide (22g, 67%) which was used without further purification.

Step D

4-(2′-chloro-4′-trifluoromethyl-6′-fluorophenoxy)-2-amino-acetanilide (22g) was stirred in water (430 $cm^3$) and concentrated hydrochloric acid (28.25 $cm^3$) added, and the mixture cooled to -2°C. Sodium nitrite (9.38g) in water (90 $cm^3$) was added slowly with stirring and cooling to 0°C. When addition was complete, the mixture was stirred for a further 15 minutes at 0°C and then stirred to room temperature over 3 hours.

The mixture was extracted with ethyl acetate, the extracts washed with water, dried ($MgSO_4$), filtered and the solvent removed under vacuum to give 1-acetyl-5-(2′-chloro-4′-trifluoromethyl-6′-fluorophenoxy)-benzotriazole (13.64g) as a brown oil.

Step E

1-acetyl-5-(2′-chloro-4′-trifluoromethyl-6′-fluorophenoxy) benzotriazole (13.64g), concentrated hydrochloric acid (48 $cm^3$) and ethanol (80 $cm^3$) were stirred and heated under reflux for 3 hours. The solvent was removed under vacuum and the residue partitioned between water and ethyl acetate. The organic phase was washed with water, dried ($MgSO_4$), filtered and the solvent removed from the filtrate under vacuum. The residue was purified by flash chromatography (silica, $CHCl_3$:EtOAc 4:1) to give 5-(2′-chloro-4′-trifluoromethyl-6′-fluorophenoxy) benzotriazole (9.6g) as a buff solid.

Step F

5-(2′-chloro-4′-trifluoromethyl-6′-fluorophenoxy) benzotriazole (8.91g) was dissolved in dry DMF (92 $cm^3$) and 50% sodium hydride (1.29g) added portionwise with stirring. After 10 minutes at room temperature, the mixture was cooled in a water bath and ethyl 2-bromopropionate (3.65 $cm^3$) added. Stirring was continued for $1\frac{1}{2}$ hours, when the mixture was poured into water (380 $cm^3$) and extracted four times with ethyl acetate. The combined organic phase was washed with water, dried ($MgSO_4$) filtered and the solvent removed from the filtrate under vacuum. The residue was purified by flash chromatography (silica, $CHCl_3$:Ether, 96:4) to yield compounds:
Compound No. 10 in Table I (1.94g)
Compound No. 11 in Taable II (2.34g)
Compound No. 12 in Table III (2.42g)
as oils.

Compounds 7, 8, 9, 13, 14, 15, 37 and 42 were prepared by anlagous methods using appropriate reactants.

EXAMPLE 3

This example illustrates the preparation of compound 10 in Table I, compound 11 in Table II and compound 12 in Table III.

Step A

M-nitrophenol (14.4g) was dissolved in DMSO (47 $cm^3$) under nitrogen. The solution was stirred, heated to 80°C and potassium hydroxide pellets (6.83g) added. After $1\frac{1}{2}$ hours at 80°C, 5-chloro-3,4-difluorobenzotrifluoride (21.9g) was added dropwise with constant stirring. When the addition was completed, the mixture was stirred and heated at 140°C for 18 hours. After cooling, most of the solvent was removed under vacuum, and the residue partitioned between water and diethylether. The organic phase was separated and the aqueous layer extracted with two further portions of ether. The ether extracts were combined, washed three times with water, dried ($MgSO_4$) and filtered. The solvent was removed from the filtrate under vacuum to give 33g of brown oil which was purified by flash chromatography (silica; $CHCl_3$:hexane, 2:3) to give 25.5g of 3-(2′-chloro-4′-trifluoromethyl-6′-fluorophenoxy)nitrobenzene as a yellow oil.

Step B

3-(2′-chloro-4′-trifluoromethyl-6′-fluorophenoxy) nitrobenzene (25.5g) was dissolved in ethylene dichloride (51 $cm^3$) stirred and cooled and in a salt/ice bath and concentrated sulphuric acid (81 $cm^3$) added. The mixture was cooled to -1°C and potassium nitrate (8.45g) added portionwise with vigorous stirring at -1 to

0° C. When addition was complete the reaction mixture was stirred for 15 minutes at 0° C and then allowed to warm to room temperature whilst stirring for a further 18 hours. The mixture was poured into ice/water (400 $cm^3$) and extracted four times with dichloromethane. The combined dichloromethane extracts were dried ($MgSO_4$), filtered and solvent removed from the filtrate under vacuum to give a pale brown oil which was purified by flash chromatography (silica, hexane: EtOAc:85:15) to give 4-(2′-chloro-4′-trifluoromethyl-6′-fluorophenoxy)-1,2-dinitrobenzene (26.78g, 93%) as an oil.

Step C

5% palladium/carbon (0.28g) was suspended in methanol (38 $cm^3$) through which nitrogen gas was being passed. A solution of sodium borohydride (0.56g) in water (15 $cm^3$) was added with stirring and ice/salt bath cooling to -5° C. A solution of 4-(2′-chloro-4′-trifluoromethyl-6′-fluorophenoxy)-1,2-dinitrobenzene (0.934g) in methanol (20 $cm^3$) was added, maintaining the stirred mixture at 0° C. When addition was complete, the cooling bath was removed and the mixture stirred at room temperature for 45 minutes. The reaction mixture was filtered through 'hyflo' and solvents removed from the filtrate under vacuum. The residue was dissolved in chloroform, washed with water and the chloroform solution dried, ($MgSO_4$) filtered and solvent removed from the filtrate under vacuum. The residue was purified by flash chromatography (silica, $CHCl_3$:EtOAc, 1:1) to give 4-(2′-chloro-4′-trifluoromethyl-6′-fluorophenoxy)-1,2-diam inobenzene (0.68g, 86%) as a pale brown oil.

Step D

4-(2′-chloro-4′-trifluoromethyl-6′-fluorophenoxy)-1,2-diaminobenzene (4.15g) was added to water (90 $cm^3$) containing concentrated hydrochloric acid (6.1 $cm^3$) with stirring. The mixture was cooled to 0° C. A solution of sodium nitrite (2.013g) in water (20 $cm^3$) was added dropwise with stirring and cooling between 0 and 5° C. During the course of the addition more water (60 $cm^3$) was added. The mixture was left to stir to room temperature overnight, extracted three times wih ethyl acetate and the combined organic phase washed with water, dried ($MgSO_4$), filtered and the solvent removed from the filtrate under vacuum. The residue was purified by flash chromatography (silica, $CHCl_3$: EtOAc, 4:1) to give 5-(2′-chloro-4′-trifluoromethyl-6′-fluorophenoxy)-benzotriazole (3.87g, 90%) as a buff solid.

Step E

5-(2′-chloro-4′-trifluoromethyl-6′-fluorophenoxy) benzotriazole was converted to compound 10 of Table I, compound 11 of Table II and compound 12 of Table III by the process described in Example 2, Step F.

Compounds 16, 17, 18, 19, 20, 21, 22, 23, 24, 25, 26, 27, 28, 29, 30, 47, 52 and 73 were prepared by analagous methods using appropriate reactants.

EXAMPLE 4

This Example describes the preparation of compound 39 of Table III.

Step A

DL 2-amino-1-propanol (5.01g) was dissolved in dry DMF (100ml) and dry potassium carbonate (13.7g) added. 4-(2′-chloro-4′trifluoromethyl-6′-fluorophenoxy)-1,2-dinit robenzene (25.05g) was added with stirring and the mixture then stirred for 18 hours at room temperature. The reaction mixture was poured into water (450 $cm^3$) and extracted three times with ethyl acetate. The combined organic extracts were washed three times with water, dried ($MgSO_4$), filtered and the solvent removed from the filtrate under vacuum. The residue was purified by flash chromatography (silica, hexane: EtOAc, 85:15) to give DL 2-[5-(2-chloro-4-trifluoromethyl-6-fluorophenoxy)-2-nitroanilino] propan-1-ol (21.4g, 75%) as a bright yellow oil.

Step B

5% palladium/C (0.11g) was added to methanol (18 $cm^3$) which was stirred and purged with nitrogen. A solution of sodium borohydride(0.22g) in water (6.2 $cm^3$) was added and the mixture cooled in ice whilst a solution of DL 2-[5-(2-chloro-4-trifluoromethyl-6-fluorophenoxy)-2-nitroanilino]propan-1-ol, (0.8g) in methanol (16 $cm^3$) was added slowly. When addition was complete the reaction was stirred for a further 15 minutes with cooling followed by 30 minutes at room temperature. The mixture was filtered through 'hyflo' and the solvent removed from the filtrate under vacuum. The residue was dissolved in chloroform and the solution washed with water. The organic phase was dried and the solvent removed under vacuum to give brown oil (0.74g, 99%) which solidified on standing. The solid was recrystallised from toluene to give DL 2-[5-(2-chloro-4-trifluoromethyl-6-fluorophenoxy)-2-amino-anilino] propan-1-ol, (0.54g, 73%).

Step C

DL 2-[5-(2-chloro-4-trifluoromethyl-6-fluorophenoxy)-2-aminoanilino] propan-1-ol, (0.519g) was added to a stirred, cooled (0 to 5°C) mixture of water (10 $cm^3$) and concentrated hydrochloric acid (0.63 $cm^3$). A solution of sodium nitrate (0.21g) in water (2.1 $cm^3$) was added dropwise, maintaining the temperature between 0 and 5°C. When the addition was completed, the reaction was kept at 0 to 5°C for 10 minutes and then stirred at room temperature over 1.5 hours. The reaction mixture was extracted three times with ethyl acetate, the organic extracts combined and washed three times with water. The organic phase was dried ($MgSO_4$), filtered and the solvent removed from the filtrate under vacuum. The residue was purified by flash chromatography (silica, $CHCl_3$:EtOAc, 7:3) to give compound 39 of Table III, DL 2-[6-(2-chloro-4-trifluoromethyl-6-fluorophenoxy)benzotriazol-1-yl] propanol (0.41g, 77%) as an oil.

EXAMPLE 5

This Example illustrates the preparation of compound No 15 in Table III.

DL 2-[6-(2-chloro-4-trifluoromethyl-6-fluorophenoxy) benzotriazol-1-yl]propanol (0.4g) was dissolved in acetone (15 $cm^3$) and the solution cooled in an ice bath. 0.62 $cm^3$ of 2.67M Jones reagent (made from 2.67g $CrO_3$ + 2.63 $cm^3$ concentrated $H_2SO_4$ + 4 $cm^3$ water diluted to 10 $cm^3$) was added in six equal portions over 30 minutes, each portion being followed by acetone (5 $cm^3$). When the additions were completed, the mixture was stirred and cooled for a further 15 minutes and then stirred at room temperature for 1 hour. Isopropanol (0.1 $cm^3$) was added followed by stirring for 10 minutes. Water (15 $cm^3$) was added and solvent removed under vacuum to leave about 20 $cm^3$. The residue was extracted three times with ethyl acetate, the organic extracts combined, washed with water, dried ($MgSO_4$), filtered and the solvent removed from the filtrate under vacuum to give crude Compound No 15 in Table III, DL 2-[6-(2-chloro-4-trifluoromethyl-6-fluorophenoxy)benzotriazol-1-yl]propionic acid (0.4g).

EXAMPLE 6

This Example illustrates the preparation of Compound No 12 in Table III.

DL 2-[6-(2-chloro-4-trifluoromethyl-6-fluorophenoxy)benzotriazol-1-yl]propionic acid (2.3g) was suspended in dry 1,2-dichloroethane (43 $cm^3$). Dry ethanol (0.37 $cm^3$) and DMAP (0.07g) were added. The stirred mixture was cooled in ice and DCC (1.18g) added. The reaction was stirred with cooling for 1 hour and then stirred to room temperature for 16 hours. The mixture was filtered and the solvent removed from the filtrate under vacuum. The residue was purified by flash chromatography (silica, $CHCl_3$:$Et_2O$, 96:4) to yield Compound 12 in Table III, DL ethyl 2-[6-(2-chloro-4-trifluoromethyl-6-fluorophenoxy) benzotriazol-1-yl]-propionate, (1.64g, 67%) as a colourless oil.

Compound 54 was prepared by an analagous method using appropriate reactants.

EXAMPLE 7

This Example illustrates the preparation of Compound No 40 in Table III.

Dl 2-[6-(2-chloro-4-trifluoromethyl-6-fluorophenoxy)benzotriazol-1-yl] propionic acid, (0.5g), 1,1-dimethyl hydrazine, (0.094 $cm^3$) and DCC (0.256 g) were dissolved in dry, stirred, ice cooled dichloromethane (5 $cm^3$). The mixture was stirred at 0 to 5° C for 1 hour and then stirred to room temperature overnight. The mixture was filtered and solvent removed from the filtrate under vacuum. The residue was purified by flash chromatography, (silica, $CHCl_3$:EtOH, 95:5) to give Compound 40 in Table III, DL 2-[6-(2-chloro-4-trifluoromethyl-6-fluorophenoxy)benzotriazol-1-yl] propionyl dimethylhydrazide (0.35g, 63%).

EXAMPLE 8

This Example illustrates the preparation of Compound 41 in Table III.

DL 2-[6-(2-chloro-4-trifluoromethyl-6-fluorphenoxy)benzotriazol-1-yl] propionyl dimethyl hydrazide (0.33g) was dissolved in methanol (12 $cm^3$) and methyl iodide (1 $cm^3$) added. The mixture was kept in the dark at room temperature for 18 days, when the solvent was removed under vacuum. The residue was triturated with ether, the ether decanted and the residue dried under vacuum to give Compound 41 in Table III, DL 2-[6-(2-chloro-4-trifluoromethyl-6-fluorophenoxy)benzotriazol-1-y] propionyl trimethyl hydrazinium iodide (0.245g).

EXAMPLE 9

This Example illustrates the preparation of Compound 81.

Ethyl 2-[6-(2-chloro-4-trifluoromethyl-6-fluorophenoxy) benzotriazol-1-yl]propionate (0.53g) was dissolved in 1,2-dichloroethane (1.5 $cm^3$) and stirred with meta-chloro-per-benzoic acid (0.28g) at room temperature for 7 days. The solvent was removed under vacuum and the residue purified by flash chromatography (silica, $CHCl_3$:EtOH, 98:2) to give Compound 43, ethyl 2-[6-(2-chloro-4-trifluoromethyl-6-fluorophenoxy) benzotriazol-1-yl-3-N-oxide] propionate (0.37g, 67%).

NMR ($CDCl_3$): δ 7.97(d)1H; 7.64(s)1H; 7.47(dd)1H); 7.06(dd)1H; 6.78(s)1H; 5.15(q) 1H; 4.17(q)2H; 1.92(d)3H; 1.17(t)3H.

EXAMPLE 10

This Example illustrates the preparation of Compound 82.

Ethyl 2-[6-(2-chloro-4-trifluoromethyl-6-fluorophenoxy) benzotriazol-1-yl]propionate (0.26g) was dissolved in dry dichloromethane (1 $cm^3$) and triethyloxonium tetrafluoroborate (0.6 $cm^3$ of 1M solution in $CH_2Cl_2$) added with stirring and left at room temperature for 18 days. The solvent was removed under vacuum to give a white solid which was washed with ether and filtered to give Compound 44, ethyl 2-[6-(2-chloro-4-trifluoromethyl- 6-fluorophenoxy-3-ethylbenzotriazol-1-ylium] propionate tetrafluoroborate (0.085g), m.p. 130.1-131.6° C.

EXAMPLE 11

This Example illustrates the preparation of Compound No 15 in table III.

Ethyl 2-[6-(2-chloro-4-trifluoromethyl-6-fluorophenoxy) benzotriazol-1-yl] propionate (0.703g) in THF (2.2 $cm^3$) and isopropanol (6.3 $cm^3$) was stirred at room temperature with a solution of sodium hydroxide (0.07g) in water (1 $cm^3$) for $2\frac{1}{2}$ hours. The solvents were removed under vacuum and the residue dissolved in water (25 $cm^3$). 2M hydrochloric acid (0.86 $cm^3$) was added with stirring. The precipitate was filtered, washed with water, air dried and recrystallised from toluene to give Compound 15 in Table III, 2-[6-(2-chloro-4-trifluoromethyl-6-fluorophenoxy) benzotriazol-1-yl] propionic acid, (0.59g, 90%) as a white solid, m.p. 173-173.9° C.

## EXAMPLE 12

This Example illustrates the preparation of Compound 38 in Table III.

DL 2-[6-(2-chloro-4-trifluoromethyl-6-fluorophenoxy) benzotriazol-1-yl]propionic acid, (0.199g) in dry 1,2-dichloroethane (4.7 $cm^3$) was stirred and refluxed with thionyl chloride (0.06g) for $1\frac{1}{2}$ hours and left to cool overnight. DMAP (0.066g) was added, the mixture stirred at room temperature for 10 minutes, when methanesulphonamide (0.05g) was added and the mixture stirred at room temperature for 2 hours. The solvent was removed under vacuum and the residue purified by flash chromatography (silica, $CHCl_3$:acetone:HOAc. 90:10:5) to give DL 2-[6-(2-chloro-4-trifluoromethyl-6-fluorophenoxy) benzotriazol-1-yl]-N-(methylsulphonyl) propionamide (0.138g, 58%) as a white solid.

Compounds 55, 56 and 57 were prepared by analagous methods using appropriate rectants.

## EXAMPLE 13

This Example illustrates the preparation of compound No 15 in Table III.

### Step A

4-(2-chloro-4-trifluoromethyl-6-fluorophenoxy)-1,2-dinitrobenzene (8g) was dissolved in dry DMF (16 $cm^3$) and DL alanine ethyl ester hydrochloride (3.24g) added followed by triethylamine (5.84 $cm^3$). The mixture was stirred for 16 hours at room temperature, poured into water (80 $cm^3$) and extracted four times with ethyl acetate. The compound extracts were washed with water, dried ($MgSO_4$), filtered and the solvent removed from the filtrate under vacuum. The residue was purified by flash chromatography ($SiO_2$, $CHCl_3$:hexane 4:1, followed by $CHCl_3$) to give DL ethyl 2-[5-(2-chloro-4-trifluoromethyl-6-fluorophenoxy)-2-nitroanilino]propionate, (4.75g), as a bright yellow oil.

### Step B

DL ethyl 2-[5-(2-chloro-4-trifluoromethyl-6-fluorophenoxy)-2-nitroanilino]propionate, (4.75g) was dissolved in THF (18 $cm^3$)/isopropanol (55 $cm^3$) and a solution of sodium hydroxide (0.86g) in water (18 $cm^3$) added slowly with stirring. The mixture was stirred at room temperature for 30 minutes and then the solvents were removed under vacuum. The residue was dissolved in water (25 $cm^3$) and added slowly to a stirred, ice cooled mixture of 5% palladium/charcoal (0.1g) in water (30 $cm^3$) and sodium borohydride (0.8g) in water (20 $cm^3$) under a nitrogen atmosphere. When addition was complete the mixture was stirred at room temperature for $2\frac{1}{2}$ hours. The reaction mixture was filtered through 'hyflo' and the filtrate cooled to 0 °C when 2M hydrochloric acid (31.6 $cm^3$) was added with stirring and cooling. A solution of sodium nitrite (1.68g) in water (10 $cm^3$) was added dropwise with stirring and cooling to 0 °C. After the addition, the mixture was stirred to room temperature for 2 hours. The mixture was extracted three times with ethyl acetate and the combined extracts washed with water, dried ($MgSO_4$), filtered and the solvent removed from the filtrate under vacuum. The residue was purified by flash chromatography ($SiO_2$, $CHCl_3$:acetone:glacial, acetic acid 85:10:5) to give DL 2-[6-(2-chloro-4-trifluoromethyl-6-fluorophenoxy) benzotriazol-1-yl] propionic acid, (1.63g).

Compounds 61, 62, 63 and 64 were prepared by analagous methods using appropriate reactants.

## EXAMPLE 14

This Example illustrates the preparation of compound No 78 and 79.

### Step A

A mixture of ethyl 2-(5- and 6-methoxybenzotriazol-1-yl) propionate (A Biodo, I Vazzana, F Sparatore; Studi Sassararesi, Sezione 2, 787, 57, 1979), (8.8g), was dissolved in dry dichloromethane (150 $cm^3$), stirred and cooled to -70° C. Boron tribromide (24 $cm^3$), in dry dichloromethane (20 $cm^3$) was added dropwise over 30 minutes, the mixture maintained at -60° C for 30 minutes and then stirred to room temperature overnight. The mixture was cooled to 4° C, ethanol (80 $cm^3$) added dropwise with stirring over 30 minutes, allowed to warm to room temperature, and the solvents removed under vacuum. Ethanol (200 $cm^3$) and concentrated sulphuric acid (2 ml) were added to the residue and the solution heated under reflux for 2 hours. The solvent was removed under vacuum and the residue dissolved in ethyl acetate, washed with water, dried ($MgSO_4$), filtered and the ethyl acetate removed under vacuum. The crude product, 7.84g, was purified by chromatography ($SiO_2$, Hexane: EtOAc, 2:3) to give 6.67g of a mixture of ethyl 2-(5- and 6-hydroxybenzotriazol-1-yl) propionate as a pale yellow oil.

Characterising data:

($CDCl_3$) $\delta$ 1.2(t,3); $\delta$ 2.0(t,3); $\delta$ 4.2(q,2), $\delta$ 5.5-5.7(m,1); $\delta$ 5.85(broads); $\delta$ 6.18(broads); $\delta$ 6.89(d); $\delta$ 6.96(dd); $\delta$ 7.13(dd); $\delta$ 7.40(m); $\delta$ 7.90(d);

Step B

A mixture of ethyl 2-(5- and 6-hydroxybenzotriazol-1-yl propionate (0.5g), anhydrous potassium carbonate (0.44g), dry methylethyl ketone (12 $cm^3$) and octafluorotoluene (1g) were heated under reflux for 0.75 hour, filtered and the filtrate evaporated under reduced pressure to give 0.93g of crude product as pale yellow oil. The crude product was purified by preparative plate chromatography ($SiO_2$, hexane:t-butylmethylether, 7:3) to give ethyl 2-(6-heptafluorotolyloxybenzotriazol-1-yl) propionate (0.4g) and ethyl 2-(5-heptafluorotolyloxybenzotriazol-1-yl) propionate (0.22g) as colourless oils.

Compounds 65, 66, 67, 68, 69, 70, 71, 72, 75, 76, 77, 80, 83, 84, 85 and 86 were prepared by analogous method using appropriate reactants.

EXAMPLE 15

This Example illustrates the preparation of compound No 44 in Table I, compound No. 49 in Table II and compound No 58 in Table III.

5-(2′-chloro-4′-trifluoromethyl-6′-fluorophenoxy benzotriazole (as prepared in Example 1 Step F) (1g) was added to pyridine (1 $cm^3$). Ethyl acrylate (0.33g) was added followed by sodium methoxide and the reaction mixture stirred at 50° C for 7 hours. The solvent was removed under vacuum and the residue partitioned between water and ethyl acetate. The organic extract was washed three times with water and dried ($MgSO_4$). The solvent was removed under vacuum leaving a brown oil. The oil was purified by flash chromatography using $SiO_2$ hexane : TBME 65:35 as eluent. Three products were obtained which were shown by NMR to be compound 44 in Table I, compound 49 in Table II and compound 58 in Table III.

Compounds 45, 46, 50, 51, 59 and 60 were prepared by analagous methods using appropriate reactants.

EXAMPLE 16

This Example illustrates the preparation of compound No. 74 in Table III.

Oxalyl chloride (0.49$cm^3$) was dissolved in dry dichloromethane (12$cm^3$) and dry DMSO in dry dichloromethane (1.25$cm^2$) was added dropwise with stirring and cooling to -50 to -60° C. DL 2-[6-(2-chloro-4-trifluoromethyl-6-fluorophenoxy)benzotriazol-1-yl] propanol (1g) in dry DMSO (5$cm^3$) was added at -50° C and stirred at -50° C for 30 minutes. Triethylamine (1.79$cm^3$) was added at -50° C and the mixture allowed to attain room temperature followed by 30 minutes stirring at room temperature. Water (25$cm^3$) was added, the organic phase separated and washed with water, dried and filtered and the organic phase removed from the filtrate under vacuum. The residue was purified by flash chromatography ($SiO_2$; hexane : TBME, 1:1 increasing to 100% TBME) to give DL 2-[6-(2-chloro-4-trifluoromethyl-6-fluorophenoxy)benzotriazol-1-yl] propionaldehyde (0.1g) as an oil.

Biological Data

The herbicidal activity of the compounds was tested as follows:

Each compound in the appropriate concentration was incorporated into a 4% emulsion of methyl cyclohexane and a 0.4% blend of 3.6 parts Tween 20 and 1 part Span 80. Tween 20 is a Trade Mark for a surface active agent comprising a condensate of 20 molar proportions of ethylene oxide with sorbitan laurate. Span 80 is a Trade mark of a surface-active agent comprising sorbitan monolaurate. Formulation was effected by dissolving the compound in the requisite amount of solvent/surfactant blend. If necessary glass beds were added, the total liquid volume adjusted to 5 ml with water and the mixture shaken to effect complete dissolution of the compound. The formulation so prepared, after removal of beads where necessary, was then diluted to final spray volume (45 ml) with water.

The spray compositions so prepared were sprayed onto young pot plants (post-emergence test) at a rate equivalent to 1000 litres per hectare. Damage to plants was assessed 13 days after spraying by comparison with untreated plants, on a scale of 0 to 5 where 0 is 0-10% damage, 1 is 11 to 25% damage, 2 is 26-50% damage, 3 is 51-80% damage, 4 is 81-95% damage and 5 is 96-100% damage.

In a test carried out to detect pre-emergence herbicidal activity, seeds of the test species were placed on the surface of plastic trays of compost and sprayed with the compositions at the rate of 1000 litres per hectare. The seeds were then covered with further compost. 20 days after spraying, the seedlings in the sprayed plastic trays were compared with the seedlings in unsprayed control trays, the damage being assessed on the same scale of 0 to 5.

The results of the tests are given in Table IV below:

TABLE IV

| COMPOUND NO | RATE OF APPLICATION kg/ha | PRE- OR POST-EMERGENCE APPLICATION | Sb | Rp | Ct | Sy | Mz | Ww | Rc | Bd | Ip | Am | Pi | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| | | | TEST PLANTS (see Table V) | | | | | | | | | | | | | | | | | | | | | | | | |
| 1 | 3 | Post | 3 | 4 | 5 | 4 | 2 | 2 | 1 | 5 | 3 | 5 | 3 | 4 | 4 | - | 5 | 4 | 2 | 2 | 1 | 1 | 2 | 0 | 1 | 0 | 1 |
| 2 | 4 | Pre | 0 | 0 | 1 | 1 | 3 | 2 | 0 | 0 | 1 | 0 | 0 | 0 | 1 | 0 | - | 0 | 0 | 3 | 4 | 3 | 3 | 4 | 1 | - | 0 |
| | | Post | 3 | 3 | 4 | 3 | 4 | 1 | 3 | 3 | 2 | 4 | 2 | 2 | 4 | - | 3 | 3 | 1 | 3 | 5 | 3 | 4 | 4 | 3 | 1 | 0 |
| 3 | 4 | Pre | 3 | 5 | 4 | 5 | 3 | 1 | 3 | 2 | 4 | 3 | 1 | 1 | 4 | 3 | - | 3 | 0 | 1 | 0 | 1 | 3 | 4 | 2 | - | 4 |
| | | Post | 3 | 5 | 5 | 4 | 3 | 2 | 1 | 5 | 5 | 5 | 5 | 5 | 5 | - | 5 | 5 | 2 | 2 | 4 | 2 | 4 | 3 | 3 | 1 | 3 |
| 4 | 3 | Pre | 1 | 0 | 0 | 0 | 2 | 0 | 0 | 1 | 0 | 0 | 0 | 0 | - | 0 | - | 0 | 0 | 0 | 0 | 0 | 0 | 0 | 0 | - | 0 |
| | | Post | 2 | 5 | 3 | 3 | 1 | 0 | 2 | 2 | 3 | 3 | 0 | 3 | 3 | - | 1 | 3 | 0 | 0 | 0 | 0 | 1 | 1 | 0 | 1 | 0 |
| 5 | 3.3 | Pre | 0 | 0 | 0 | 0 | 0 | 0 | 0 | 0 | 0 | 0 | 0 | 0 | - | 0 | - | 0 | 0 | 0 | 0 | 0 | 0 | 0 | 0 | - | 1 |
| | | Post | 2 | 2 | 2 | 1 | 1 | 2 | 0 | 2 | 2 | 3 | 0 | 0 | 3 | - | 0 | 3 | 1 | 1 | 1 | 1 | 2 | 2 | 2 | 3 | 1 |
| 6 | 3 | Pre | 0 | 2 | 1 | 0 | 4 | 0 | 1 | 4 | 4 | 3 | 0 | 0 | - | 3 | - | 0 | 0 | 0 | 0 | 0 | 1 | 0 | 0 | - | 0 |
| | | Post | 3 | 5 | 4 | 4 | 0 | 0 | 1 | 5 | 5 | 5 | 3 | 5 | 5 | - | 5 | 5 | 1 | 0 | 0 | 0 | 3 | 3 | 2 | 0 | 2 |

TABLE IV (Contd.)

| COMPOUND NO | RATE OF APPLICATION kg/ha | PRE- OR POST-EMERGENCE APPLICATION | TEST PLANTS (see Table V) Sb | Rp | Ct | Sy | Mz | Ww | Rc | Bd | Ip | Am | Pi | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| 7 | 3.5 | Pre | 0 | 3 | 1 | 2 | 1 | 0 | 1 | 4 | 3 | 5 | 2 | 3 | 3 | 0 | - | 2 | 2 | 1 | 0 | 1 | 4 | 0 | 2 | - | 2 |
| | | Post | 5 | 5 | 4 | 4 | 4 | 3 | 3 | 5 | 5 | 5 | 5 | 5 | 5 | - | 5 | 5 | 3 | 3 | 3 | 4 | 4 | 3 | 3 | 3 | 3 |
| 8 | 4 | Pre | 1 | 3 | 1 | 1 | 0 | 1 | 3 | 4 | 1 | 5 | 1 | 4 | 2 | 0 | - | 3 | 1 | 1 | 2 | 2 | 3 | 0 | 1 | - | 0 |
| | | Post | 3 | 5 | 4 | 3 | 5 | 4 | 2 | 4 | 5 | 5 | 5 | 4 | 5 | - | 4 | 5 | 3 | 3 | 5 | 3 | 5 | 5 | 5 | 4 | 2 |
| 9 | 3.5 | Pre | 5 | 5 | 4 | 5 | 5 | 4 | 5 | 5 | 5 | 5 | 5 | 5 | 5 | 5 | - | 5 | 5 | 5 | 5 | 5 | 5 | 5 | 4 | - | 5 |
| | | Post | 5 | 5 | 5 | 5 | 5 | 4 | 5 | 5 | 5 | 5 | 5 | 5 | 5 | - | 5 | 5 | 5 | 5 | 5 | 5 | 5 | 5 | 5 | 5 | 4 |
| 10 | 1 | Pre | 0 | 0 | 1 | 2 | 1 | 0 | 0 | 1 | 0 | 1 | 0 | 0 | 0 | 0 | - | 0 | 0 | 0 | 0 | 0 | 2 | 0 | 1 | - | 1 |
| | | Post | 5 | 5 | 5 | 4 | 5 | 3 | 3 | 5 | 3 | 5 | 5 | - | 5 | - | 4 | 4 | 1 | 3 | 3 | 3 | 4 | 3 | 4 | 2 | 1 |
| 11 | 2.89 | Pre | 1 | 2 | 0 | 1 | 2 | 0 | 0 | 3 | 3 | 4 | 0 | 5 | 3 | 0 | - | 5 | 0 | 0 | 2 | 0 | 2 | 1 | 0 | - | 0 |
| | 1 | Post | 4 | 5 | 5 | 4 | 4 | 2 | 2 | 5 | 5 | 5 | 5 | - | 5 | - | 5 | 5 | 5 | 2 | 4 | 2 | 4 | 5 | 5 | 3 | 2 |
| 12 | 1 | Pre | 5 | 5 | 3 | 5 | 5 | 5 | 5 | 5 | 5 | 5 | 5 | 5 | 5 | 5 | - | 5 | 5 | 5 | 5 | 5 | 5 | 5 | 5 | - | 4 |
| | | Post | 5 | 5 | 5 | 5 | 5 | 5 | 5 | 5 | 5 | 5 | 5 | - | 5 | - | 5 | 5 | 5 | 5 | 5 | 5 | 5 | 5 | 5 | 5 | 3 |

TABLE IV (Contd.)

| COMPOUND NO | RATE OF APPLICATION kg/ha | PRE- OR POST-EMERGENCE APPLICATION | Sb | Rp | Ct | Sy | Mz | Ww | Rc | Bd | Ip | Am | Pi | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| | | | TEST PLANTS (see Table V) | | | | | | | | | | | | | | | | | | | | | | | | |
| 13 | 1 | Pre | 0 | 0 | 0 | 1 | 0 | 0 | 0 | 0 | 0 | 0 | 0 | 0 | 0 | 0 | - | 0 | 0 | 0 | 0 | 0 | 0 | 0 | 0 | - | 0 |
| | | Post | 1 | 3 | 3 | 3 | 3 | 1 | 0 | 1 | 3 | 4 | 3 | - | 1 | - | 2 | 2 | 1 | 1 | 1 | 1 | 1 | 1 | 2 | 0 | 0 |
| 14 | 1 | Pre | 4 | 4 | 0 | 1 | 0 | 0 | 1 | 4 | 4 | 5 | 3 | 4 | 4 | 0 | - | 4 | 0 | 0 | 3 | 0 | 3 | 0 | 3 | - | 0 |
| | | Post | 2 | 5 | 5 | 3 | 4 | 1 | 1 | 4 | 5 | 5 | 4 | - | 5 | - | 5 | 2 | 1 | 5 | 0 | 4 | 4 | 4 | 5 | 3 | 1 |
| 15 | 1 | Pre | 5 | 5 | 5 | 5 | 5 | 5 | 5 | 5 | 5 | 5 | 5 | 5 | 5 | 5 | - | 5 | 5 | 5 | 5 | 5 | 5 | 5 | 5 | - | 5 |
| | | Post | 5 | 5 | 5 | 5 | 5 | 5 | 5 | 5 | 5 | 5 | 5 | - | 5 | - | 5 | 5 | 5 | 5 | 5 | 5 | 5 | 5 | 5 | 4 | 5 |
| 31 | 2.8 | Pre | 0 | 0 | 0 | 0 | 0 | 0 | 3 | 0 | 0 | 0 | 0 | 0 | 0 | 0 | - | 0 | 0 | 0 | 3 | 0 | 0 | 0 | - | - | - |
| | | Post | 2 | 2 | 2 | 2 | 2 | 0 | 0 | 2 | 4 | 3 | 3 | 2 | 2 | - | 2 | 2 | 0 | 0 | 2 | 0 | 3 | 2 | 2 | 0 | 0 |
| 32 | 3.57 | Pre | 0 | 0 | 0 | 1 | 3 | 3 | 5 | 0 | 2 | 0 | 0 | 0 | 0 | 0 | - | 1 | 0 | 1 | 5 | 4 | 5 | 4 | - | - | - |
| | | Post | 2 | 3 | 3 | 3 | 4 | 3 | 3 | 1 | 4 | 3 | 0 | 3 | 5 | - | 3 | 4 | 3 | 3 | 4 | 3 | 5 | 5 | 4 | 2 | 2 |

TABLE IV (Contd.)

| COMPOUND NO | RATE OF APPLICATION kg/ha | PRE- OR POST-EMERGENCE APPLICATION | Sb | Rp | Ct | Sy | Mz | Ww | Rc | Bd | Ip | Am | Pi | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| | | | TEST PLANTS (see Table V) | | | | | | | | | | | | | | | | | | | | | | | | |
| 33 | 2.2 | Pre | 3 | 5 | 3 | 3 | 4 | 4 | 4 | 0 | 4 | 3 | 3 | 3 | 3 | 0 | - | 0 | 2 | 2 | 5 | 2 | 5 | 5 | - | - | - |
| | | Post | 4 | 5 | 5 | 5 | 4 | 3 | 2 | 5 | 5 | 5 | 5 | 5 | 5 | - | 5 | 5 | 4 | 3 | 4 | 2 | 5 | 5 | 5 | 2 | 3 |
| 37 | 0.25 | Pre | 5 | 5 | 3 | 4 | 4 | 4 | 4 | 5 | 5 | 5 | 4 | 5 | 5 | 5 | - | 5 | 3 | 2 | 5 | 4 | 5 | 5 | 4 | - | 1 |
| | | Post | 5 | 5 | 5 | 5 | 5 | 5 | 4 | 5 | 5 | 5 | 5 | 5 | 5 | - | 5 | 5 | 5 | 5 | 5 | 5 | 5 | 5 | 5 | 5 | 3 |
| 38 | 1 | Pre | 5 | 5 | 4 | 4 | 4 | 3 | 5 | 5 | 5 | 5 | 5 | 5 | 5 | 5 | - | 5 | 4 | 2 | 5 | 3 | 5 | 5 | 5 | - | 5 |
| | | Post | 5 | 5 | 5 | 5 | 5 | 3 | 3 | 5 | 5 | 5 | 5 | 5 | 5 | - | 5 | 5 | 5 | 3 | 5 | 3 | 5 | 5 | 5 | 3 | 3 |
| 39 | 1 | Pre | 5 | 5 | 5 | 4 | 4 | 4 | 5 | 5 | 5 | 5 | 5 | 5 | 5 | 3 | - | 5 | 5 | 5 | 5 | 5 | 5 | 5 | 5 | - | 4 |
| | | Post | 5 | 5 | 5 | 5 | 5 | 4 | 3 | 5 | 5 | 5 | 5 | 5 | 5 | - | 5 | 5 | 5 | 4 | 5 | 4 | 5 | 5 | 5 | 4 | 3 |

TABLE V

Abbreviations used for Test Plants

Sb - Sugar beet
Rp - Rape
Ct - Cotton
Sy - Soybean
Mz - Maize
Ww - Winter wheat
Rc - Rice
Bd - *Bidens pilosa*
Ip - *Ipomoea purpurea*
Am - *Amaranthus retroflexus*
Pi - *Polygonum aviculare*
Ca - *Chenopodium album*
Ga - *Galium aparine*
Xa - *Xanthium spinosum*
Xs - *Santhium strumarium*
Ab - *Abutilon theophrasti*
Co - *Cassia obtusifolia*
Av - *Avena fatua*
Dg - *Digitaria sanguinalis*
Al - *Alopecurus myosuroides*
St - *Setaria viridis*
Ec - *Echinoohloa crus-galli*
Sh - *Sorghum halepense*
Ag - *Agropyron repens*
Cn - *Cyperus rotundus*

The herbicidal activity of some of the compounds was tested by an alternative method as follows:

Each compound in the appropriate concentration was incorporated into a 4% emulsion of methyl cyclohexanone and 0.4% blend of 3.6 parts Tween 20 and 1 part Span 80. Tween 20 is a Trade Mark for a surface active agent comprising a condensate fo 20 molar proportions of ethylene oxide with sorbitan laurate. Span 80 is a Trade Mark for a surface-active agent comprising sorbitan monolaurate. Formulation was effected by dissolving the compound in the requisite amount of solvent/surfaotant blend. If necessary glass beads were added, the total liquid volume adjusted to 5 ml with water and the mixture shaken to effect complete dissolution of the compound. The formulation so prepared, after removal of beads where necessary, was then diluted to final spray volume (45 ml) with water.

The spray compositions so prepared were sprayed onto young pot plants (post-emergence test) at a rate equivalent to 1000 litres per hectare. Damage to plants was assessed 13 days after spraying by comparison with untreated plants, on a scale of 0 to 9 where 0 is 0% damage, 1 is 1-5% damage, 2 is 6-15% damage, 3 is 16-25% damage, 4 is 26-35% damage, 5 is 36-59% damage, 6 is 60-69% damage, 7 is 70-79% damage, 8 is 80-89% damage and 9 is 90-100% damage.

In a test carried out to detect pre-emergence herbicidal activity, Crop seeds were sown at 2 cm depth (i.e. Sb, Ct, Rp, Ww, Mz, Rc, Sy) and weed seeds at 1 cm depth beneath compost and sprayed with the compositions at the rate of 1000 litres per hectare. 20 days after spraying, the seedlings in the sprayed plastic trays were compared with the seedlings in unsporayed control trays, the damage being assessed on the same scale of 0 to 9.

The results of the tests are given in Table VI below.

TABLE VI

| COMPOUND NO | RATE OF APPLICATION kg/ha | PRE- OR POST-EMERGENCE APPLICATION | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
|---|---|---|---|---|---|---|---|---|---|---|---|---|---|---|---|---|---|---|---|---|---|---|---|---|---|---|---|
| | | | TEST PLANTS (see Table VII) | | | | | | | | | | | | | | | | | | | | | | | | |
| 16 | 1 | Pre | 0 | 0 | 0 | 0 | 0 | 0 | 0 | 0 | 0 | 0 | 0 | 0 | 0 | 5 | 0 | 0 | - | 0 | 3 | - | 2 | 0 | 0 | 0 | 0 |
| | 0.25 | Post | 2 | 7 | 8 | 4 | 2 | 0 | 0 | 5 | 9 | 2 | 9 | 2 | 8 | 9 | 9 | - | 5 | 0 | 0 | 1 | 7 | 6 | 6 | 5 | 0 |
| 17 | 1 | Pre | 8 | 7 | 5 | 0 | 0 | 0 | 0 | 0 | 9 | 0 | 9 | 0 | 8 | 2 | 0 | 0 | - | 0 | 0 | - | 7 | 0 | 0 | 0 | 1 |
| | | Post | 8 | 9 | 9 | 9 | 9 | 2 | 2 | 9 | 9 | 9 | 9 | 9 | 9 | 9 | 9 | - | 9 | 1 | 5 | 2 | 8 | 9 | 8 | 7 | 0 |
| 18 | 0.25 | Pre | 9 | 7 | 2 | 5 | 0 | 0 | 0 | 4 | 9 | 2 | 9 | 2 | 9 | 7 | 0 | 2 | - | 0 | 0 | - | 7 | 9 | 6 | 8 | 2 |
| | 0.0625 | Post | 9 | 9 | 9 | 9 | 9 | 2 | 9 | 9 | 9 | 9 | 9 | 9 | 9 | 9 | 9 | - | 9 | 5 | 5 | 1 | 8 | 9 | 9 | 9 | 3 |
| 19 | 1 | Pre | 0 | 0 | 0 | 0 | 0 | 0 | 0 | 0 | 9 | 0 | 9 | 1 | 5 | 5 | 2 | 0 | - | 0 | 3 | - | 0 | 0 | 0 | 0 | 0 |
| | 0.25 | Post | 6 | 9 | 8 | 6 | 6 | 1 | 2 | 9 | 9 | 9 | 9 | 9 | 9 | 9 | 9 | - | 9 | 1 | 0 | 2 | 8 | 2 | - | 8 | 1 |
| 20 | 1 | Pre | 0 | 0 | 0 | 0 | 0 | 0 | 0 | 0 | 0 | 0 | 0 | 0 | 0 | 0 | 0 | 0 | - | 0 | 0 | - | 6 | 0 | 0 | 0 | 0 |
| | | Post | 5 | 9 | 9 | 8 | 7 | 1 | 2 | 8 | 8 | 9 | 9 | 4 | 9 | 9 | 5 | - | 9 | 1 | 0 | 1 | 6 | 6 | 5 | 5 | 0 |
| 21 | 0.25 | Pre | 9 | 2 | 3 | 0 | 0 | 2 | 0 | 5 | 9 | 4 | 9 | 2 | 7 | 8 | 0 | 2 | - | 0 | 0 | - | 7 | 0 | 2 | 0 | 0 |
| | 0.0625 | Post | 9 | 9 | 9 | 8 | 9 | 2 | 2 | 9 | 9 | 9 | 9 | 9 | 9 | 9 | 9 | - | 9 | 3 | 4 | 2 | 8 | 7 | 8 | 8 | 1 |

TABLE VI (Contd.)

| COMPOUND NO | RATE OF APPLICATION kg/ha | PRE- OR POST-EMERGENCE APPLICATION | TEST PLANTS (see Table VII) Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
|---|---|---|---|---|---|---|---|---|---|---|---|---|---|---|---|---|---|---|---|---|---|---|---|---|---|---|---|
| 22 | 1 | Pre | 0 | 0 | 0 | 3 | 0 | 0 | 0 | 0 | 9 | 0 | 9 | 2 | 2 | 0 | 0 | 0 | - | 0 | 1 | - | 0 | 0 | 0 | 0 | 0 |
| | 0.25 | Post | 4 | 9 | 7 | 6 | 8 | 0 | 2 | 5 | 9 | 8 | 9 | 7 | 9 | 9 | 9 | - | 9 | 1 | 1 | 0 | 8 | 6 | 7 | 8 | 1 |
| 23 | 1 | Pre | 0 | 2 | 1 | 0 | 0 | 0 | 0 | 2 | 0 | 0 | 0 | 1 | 0 | 0 | 0 | 0 | - | 0 | 0 | - | 5 | 0 | 0 | 2 | 2 |
| | | Post | 4 | 9 | 9 | 3 | 6 | 1 | 1 | 5 | 6 | 9 | 9 | 3 | 8 | 9 | 5 | - | 9 | 0 | 0 | 1 | 6 | 6 | 1 | 5 | 0 |
| 24 | 0.25 | Pre | 9 | 1 | 1 | 3 | 0 | 2 | 0 | 0 | 9 | 1 | 9 | 5 | 9 | 0 | 0 | 2 | - | 0 | 0 | - | 2 | 9 | 4 | 3 | 0 |
| | 0.0625 | Post | 9 | 9 | 9 | 6 | 8 | 1 | 3 | 7 | 9 | 9 | 9 | 9 | 9 | 9 | 9 | - | 9 | 2 | 2 | 1 | 9 | 8 | 9 | 8 | 0 |
| 25 | 1 | Pre | 0 | 0 | 0 | 0 | 0 | 0 | 0 | 0 | 0 | 0 | 0 | 0 | 0 | 3 | 0 | 0 | - | 0 | 2 | - | 0 | 0 | 0 | 0 | 0 |
| | 0.25 | Post | 1 | 1 | 0 | 1 | 1 | 0 | 0 | 2 | 8 | 0 | 8 | 1 | 6 | 7 | 3 | - | 2 | 0 | 0 | 0 | 2 | 1 | 1 | 1 | 0 |
| 26 | 1 | Pre | 0 | 0 | 3 | 3 | 0 | 0 | 0 | 2 | 3 | 2 | 2 | 0 | 2 | 0 | 0 | 8 | - | 0 | 0 | - | 5 | 0 | 6 | 7 | 1 |
| | | Post | 3 | 9 | 4 | 5 | 7 | 2 | 1 | 5 | 7 | 1 | 9 | 5 | 8 | 9 | 2 | - | 5 | 1 | 1 | 0 | 8 | 3 | 3 | 6 | 0 |
| 27 | 0.25 | Pre | 2 | 0 | 2 | 0 | 3 | 0 | 0 | 4 | 9 | 2 | 9 | 5 | 5 | 2 | 0 | 0 | - | 0 | 0 | - | 4 | 0 | 0 | 0 | 0 |
| | 0.0625 | Post | 5 | 9 | 6 | 5 | 8 | 1 | 2 | 7 | 9 | 4 | 9 | 7 | 9 | 9 | 9 | - | 9 | 1 | 1 | 2 | 9 | 6 | 6 | 6 | 2 |

TABLE VI (Contd.)

| COMPOUND NO | RATE OF APPLICATION kg/ha | PRE- OR POST-EMERGENCE APPLICATION | TEST PLANTS (see Table VII) | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 28 | 1 | Pre | 0 | 1 | 3 | 3 | 0 | 0 | 0 | 0 | 0 | - | 0 | 0 | 0 | 2 | 0 | - | - | 0 | 0 | - | 0 | 0 | 4 | 0 | 0 |
| | 0.25 | Post | 5 | 2 | 3 | 9 | 6 | 2 | 2 | 4 | 7 | 5 | 7 | 2 | 9 | 9 | 7 | - | 5 | 2 | 2 | 1 | 1 | 2 | 2 | 2 | 0 |
| 29 | 1 | Pre | 0 | 0 | 0 | 0 | 1 | 9 | 0 | 0 | 8 | - | 8 | 0 | 2 | 0 | 2 | - | - | 0 | 0 | - | 0 | 0 | 0 | 0 | 0 |
| | | Post | 9 | 9 | 9 | 8 | 5 | 3 | 3 | 9 | 9 | 9 | 9 | 9 | 9 | 9 | 9 | - | 9 | 2 | 1 | 2 | 8 | 3 | 5 | 6 | 2 |
| 30 | 1 | Pre | 9 | 9 | 5 | 5 | 0 | 9 | 0 | - | 9 | - | 9 | 9 | 9 | 9 | 9 | - | - | 7 | 5 | - | 7 | 9 | 9 | 9 | 3 |
| | 0.25 | Post | 9 | 9 | 9 | 9 | 9 | 9 | 9 | 9 | 9 | 9 | 9 | 9 | 9 | 9 | 9 | - | 9 | 9 | 9 | 9 | 8 | 9 | 9 | 9 | 8 |
| 35 | 1 | Pre | 0 | 0 | 0 | 0 | 0 | 0 | 0 | - | 0 | - | 0 | 0 | 0 | 0 | 0 | 0 | - | 0 | 0 | - | 0 | 0 | 0 | 5 | - |
| | | Post | 1 | 0 | 0 | 2 | 8 | 0 | 0 | 0 | 0 | 3 | 0 | 0 | 2 | 0 | 0 | - | 0 | 0 | 5 | 0 | 0 | 6 | 0 | 1 | 0 |

TABLE VI (Contd.)

| COMPOUND NO | RATE OF APPLICATION kg/ha | PRE- OR POST-EMERGENCE APPLICATION | TEST PLANTS (see Table VII) | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 40 | 1 | Pre | 9 | 9 | 4 | 4 | 6 | - | 7 | 9 | 9 | - | 9 | 9 | 9 | 9 | 9 | - | - | 8 | 7 | - | 9 | 9 | 9 | 9 | 9 |
| | | Post | 9 | 9 | 9 | 9 | 9 | 5 | 9 | 9 | 9 | 9 | 9 | 9 | 9 | 9 | 9 | - | 9 | 9 | 9 | 6 | 7 | 9 | 9 | 9 | 9 |
| 41 | 1 | Pre | 9 | 9 | 4 | 3 | 6 | - | 5 | 9 | 9 | - | 9 | 9 | 9 | 5 | 9 | - | - | 2 | 2 | - | 9 | 9 | 8 | 8 | 2 |
| | | Post | 9 | 9 | 9 | 9 | 9 | 3 | 7 | 9 | 9 | 9 | 9 | 9 | 9 | 9 | 9 | - | 9 | 9 | 7 | 4 | 9 | 9 | 9 | 8 | 7 |
| 42 | 0.4 | Pre | 0 | 0 | 4 | 0 | 0 | 0 | 0 | 0 | 2 | 0 | 0 | 2 | 2 | 1 | 0 | 4 | - | 0 | 0 | - | 6 | 0 | 0 | 0 | 0 |
| | | Post | 3 | 8 | 5 | 7 | 8 | 2 | 1 | 6 | 7 | 2 | 6 | 2 | 9 | 8 | 3 | - | 5 | 0 | 1 | 0 | 8 | 6 | 7 | 5 | 0 |
| 43 | 1 | Pre | 9 | 9 | 5 | 2 | 4 | 5 | 5 | - | 9 | - | 9 | 9 | 9 | 9 | 9 | 5 | - | 0 | 1 | - | 9 | 9 | 9 | 8 | 1 |
| | | Post | 9 | 9 | 9 | 9 | 9 | 7 | 8 | 9 | 9 | 9 | 9 | 9 | 9 | 9 | 9 | - | 9 | 9 | 9 | 8 | 9 | 9 | 9 | 9 | 7 |
| 44 | 1 | Pre | 5 | 0 | 0 | 0 | 0 | 0 | 0 | - | 0 | - | 3 | 0 | 5 | 0 | 0 | 0 | - | 0 | 0 | - | 0 | 0 | 0 | 0 | 0 |
| | | Post | 9 | 9 | 9 | 9 | 9 | 5 | 6 | 9 | 9 | 9 | 9 | 9 | 9 | 9 | 9 | 9 | - | 9 | 5 | 6 | 9 | 4 | 9 | 9 | 4 |

TABLE VI (Contd.)

| COMPOUND NO | RATE OF APPLICATION kg/ha | PRE- OR POST-EMERGENCE APPLICATION | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
|---|---|---|---|---|---|---|---|---|---|---|---|---|---|---|---|---|---|---|---|---|---|---|---|---|---|---|---|
| 45 | 1 | Pre | 0 | 0 | 0 | 0 | 0 | 0 | 0 | - | 6 | - | 0 | 0 | 0 | 0 | 0 | 0 | - | 0 | 0 | - | 0 | 0 | 0 | 0 | 0 |
| | | Post | 7 | 9 | 2 | 8 | 1 | 1 | 0 | 0 | 9 | 7 | 9 | 1 | 3 | 8 | 9 | - | 4 | 3 | 2 | 0 | 1 | 2 | 2 | 0 | 0 |
| 46 | 1 | Pre | 0 | 0 | 0 | 0 | 0 | 0 | 0 | 0 | 0 | - | 0 | 0 | 0 | 0 | 0 | 0 | - | 0 | 0 | - | 0 | 0 | 0 | 0 | 0 |
| | | Post | - | 4 | 2 | 7 | 6 | 0 | 3 | 7 | 0 | 3 | 9 | 0 | 6 | 2 | 9 | - | 5 | 0 | 0 | 2 | 0 | 1 | 2 | 0 | 0 |
| 48 | 1 | Pre | 0 | 0 | 0 | 0 | 0 | 0 | 0 | - | 0 | - | 0 | 0 | 0 | 0 | 0 | 0 | - | 0 | 0 | - | 0 | 0 | 0 | 0 | 0 |
| | | Post | 2 | 3 | 0 | 0 | 0 | 0 | 0 | 2 | 0 | 5 | 0 | 0 | 1 | 0 | 0 | - | 0 | 0 | 0 | 0 | 0 | 0 | 0 | 0 | 0 |
| 49 | 1 | Pre | 1 | 9 | 0 | 0 | 0 | - | 0 | - | 0 | - | 5 | 2 | 0 | 0 | 0 | 0 | - | 0 | 0 | - | 0 | 0 | 0 | 0 | 0 |
| | | Post | 6 | 9 | 6 | 3 | 8 | 0 | 2 | 9 | 9 | 9 | 9 | 3 | 9 | 7 | 9 | - | 3 | 0 | 0 | 0 | 0 | 0 | 1 | 7 | 0 |
| 50 | 1 | Pre | 0 | 0 | 0 | 0 | 0 | 0 | 0 | - | 9 | - | 9 | 0 | 0 | 0 | 0 | 0 | - | 0 | 0 | - | 0 | 0 | 0 | 0 | 0 |
| | | Post | 9 | 9 | 6 | 6 | 3 | 1 | 0 | 9 | 9 | 9 | 9 | 6 | 9 | 9 | 9 | - | 7 | 0 | 0 | 0 | 0 | 0 | 0 | 0 | 0 |
| 51 | 1 | Pre | 0 | 0 | 0 | 0 | 0 | 0 | 0 | - | 0 | - | 0 | 0 | 0 | 0 | 0 | 0 | - | 0 | 0 | - | 0 | 0 | 0 | 0 | 0 |
| | | Post | 8 | 8 | 7 | 7 | 5 | 1 | 2 | 6 | 9 | 9 | 9 | 3 | 9 | 9 | 9 | - | 7 | 0 | 0 | 3 | 0 | 0 | 0 | 0 | 0 |

TEST PLANTS (see Table VII)

TABLE VI

| COMPOUND NO | RATE OF APPLICATION kg/ha | PRE- OR POST-EMERGENCE APPLICATION | TEST PLANTS (see Table VII) Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
|---|---|---|---|---|---|---|---|---|---|---|---|---|---|---|---|---|---|---|---|---|---|---|---|---|---|---|---|
| 53 | 1 | Pre | 9 | 3 | 0 | 0 | 0 | 0 | 0 | - | 4 | - | 0 | 0 | 0 | 0 | 0 | 0 | - | 0 | 0 | - | 0 | 0 | 0 | 0 | 0 |
| | | Post | 6 | 9 | 7 | 7 | 8 | 2 | 8 | 9 | 9 | 8 | 9 | 9 | 9 | 9 | 9 | - | 8 | 8 | 8 | 5 | 9 | 7 | 7 | 7 | 0 |
| 54 | 1 | Pre | 9 | 9 | 0 | 0 | 0 | 9 | 0 | - | 9 | - | 9 | 9 | 9 | 0 | 9 | 0 | - | 0 | 3 | - | 9 | 9 | 9 | 8 | 6 |
| | | Post | 9 | 9 | 7 | 5 | 9 | 2 | 4 | 3 | 9 | 9 | 9 | 7 | 9 | 9 | 9 | - | 9 | 4 | 0 | 4 | 9 | 6 | 2 | 9 | 0 |
| 55 | 1 | Pre | 9 | 9 | 8 | 7 | 3 | 3 | 8 | - | 9 | - | 9 | 9 | 9 | 7 | 9 | 7 | - | 9 | 8 | - | 9 | 9 | 9 | 9 | 2 |
| | | Post | 9 | 9 | 9 | 9 | 9 | 9 | 9 | 9 | 9 | 9 | 9 | 9 | 9 | 9 | 9 | - | 9 | 9 | 9 | 9 | 9 | 9 | 9 | 8 | 9 |
| 56 | 1 | Pre | 9 | 5 | 0 | 0 | 0 | 0 | 0 | - | 9 | - | 9 | 9 | 9 | 3 | 9 | 0 | - | 3 | 3 | - | 7 | 9 | 9 | 0 | 0 |
| | | Post | 9 | 9 | 9 | 9 | 9 | 5 | 8 | 9 | 9 | 9 | 9 | 9 | 9 | 9 | 9 | - | 9 | 8 | 4 | 8 | 9 | 9 | 9 | 7 | 9 |
| 57 | 1 | Pre | 9 | 0 | 0 | 0 | 3 | 0 | 2 | - | 9 | - | 0 | 0 | 0 | 2 | 7 | 0 | - | 0 | 0 | - | 7 | 9 | 9 | 9 | 0 |
| | | Post | 5 | 7 | 7 | 6 | 8 | 4 | 6 | 8 | 9 | 7 | 9 | 6 | 9 | 7 | 9 | - | 9 | 3 | 3 | 3 | 9 | 8 | 3 | 3 | 4 |
| 58 | 0.0625 | Pre | 0 | 0 | 1 | 1 | 0 | 0 | 1 | - | 0 | - | 0 | 0 | 0 | 0 | 0 | 0 | - | 0 | 0 | - | 0 | 0 | 0 | 0 | 0 |
| | | Post | 9 | 9 | 4 | 3 | 7 | 2 | 3 | 9 | 9 | 9 | 9 | 7 | 8 | 7 | 9 | - | 6 | 5 | 5 | 0 | 9 | 0 | 2 | 7 | 0 |

TABLE VI

| COMPOUND NO | RATE OF APPLICATION kg/ha | PRE- OR POST-EMERGENCE APPLICATION | TEST PLANTS (see Table VII) | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 61 | 1 | Pre | 9 | 9 | 4 | 3 | 2 | 9 | 7 | - | 9 | - | 9 | 9 | 9 | 9 | 9 | 7 | - | 3 | 0 | - | 9 | 9 | 9 | 9 | 2 |
| | 0.03 | Post | 9 | 9 | 4 | 9 | 9 | 6 | 9 | - | 9 | 0 | 9 | 9 | 9 | 9 | 3 | - | 9 | 2 | 5 | 8 | 8 | 5 | 4 | 9 | 2 |
| 62 | 1 | Pre | 9 | 9 | 0 | 2 | 2 | 9 | 6 | - | 9 | - | 9 | 9 | 9 | 0 | 9 | 7 | - | 5 | 0 | - | 9 | 9 | 9 | 9 | 1 |
| | 0.03 | Post | 4 | 9 | 2 | 8 | 0 | 4 | 2 | - | 9 | 1 | 9 | 3 | 7 | 5 | 6 | - | 5 | 1 | 2 | 1 | 6 | 1 | 1 | 0 | 0 |
| 63 | 1 | Pre | 9 | 9 | 7 | 0 | 0 | 9 | 5 | - | 9 | - | 9 | 9 | 9 | 9 | 9 | 7 | - | 0 | 1 | - | 9 | 9 | 9 | 9 | 6 |
| | 0.03 | Post | 9 | 9 | 8 | 9 | 7 | 9 | 9 | - | 9 | 9 | 9 | 9 | 9 | 9 | 9 | - | 9 | 6 | 6 | 7 | 9 | 9 | 8 | 9 | 6 |
| 64 | 1 | Pre | 9 | 9 | 2 | 0 | 0 | 9 | 0 | - | 9 | - | 9 | 9 | 9 | 4 | 9 | 5 | - | 0 | 0 | - | 9 | 9 | 4 | 9 | 0 |
| | 0.03 | Post | 4 | 9 | 3 | 7 | 8 | 5 | 5 | - | 9 | 9 | 9 | 7 | 9 | 8 | 9 | - | 9 | 2 | 3 | 4 | 8 | 1 | 2 | 2 | 1 |
| 65 | 1 | Pre | 0 | 0 | 0 | 0 | 0 | 0 | 0 | - | 0 | - | 0 | 0 | 0 | 0 | 0 | 0 | - | 0 | 0 | - | 0 | 0 | 0 | 0 | 0 |
| | | Post | 0 | 0 | 0 | 0 | 0 | 0 | 0 | 1 | 6 | 0 | 7 | 0 | 0 | 0 | 0 | - | 2 | 0 | 0 | 0 | - | 0 | 0 | 0 | 0 |
| 67 | 1 | Pre | 0 | 0 | 0 | 0 | 0 | 0 | 0 | 0 | 0 | - | 0 | - | 0 | 0 | 0 | 0 | - | 0 | 0 | - | 0 | 0 | 0 | 0 | 0 |
| | | Post | 3 | 9 | 5 | 9 | 5 | 4 | 5 | 9 | 9 | 8 | 9 | 3 | 9 | 9 | 8 | - | 7 | 6 | 3 | 4 | 6 | 5 | 2 | 5 | 1 |

TABLE VI

| COMPOUND NO | RATE OF APPLICATION kg/ha | PRE- OR POST-EMERGENCE APPLICATION | TEST PLANTS (see Table VII) Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
|---|---|---|---|---|---|---|---|---|---|---|---|---|---|---|---|---|---|---|---|---|---|---|---|---|---|---|---|
| 68 | 1 | Pre | 0 | 0 | 0 | 0 | 0 | 0 | 0 | 0 | 0 | - | 0 | - | 0 | 0 | 0 | 0 | - | 0 | 0 | - | 0 | 0 | 0 | 0 | 0 |
| | | Post | 3 | 4 | 1 | 1 | 0 | 0 | 3 | 0 | 0 | 0 | 9 | 0 | 2 | 2 | 9 | - | 6 | 1 | 0 | 2 | 0 | 1 | 0 | 0 | 0 |
| 69 | 1 | Pre | 0 | 0 | 0 | 0 | 0 | 0 | 0 | - | 9 | - | 9 | 4 | 0 | 2 | 0 | 0 | - | 0 | 4 | - | 3 | 7 | 0 | 8 | 0 |
| | | Post | 5 | 9 | 5 | 3 | 5 | 3 | 3 | 5 | 9 | 6 | 9 | 7 | 9 | 9 | 9 | - | 9 | 9 | 3 | 3 | 0 | 9 | 7 | 9 | 5 |
| 80 | 1 | Pre | 5 | 2 | 0 | 0 | 0 | 9 | 7 | - | 8 | - | 8 | 7 | 9 | 5 | 5 | 5 | - | 0 | 0 | - | 9 | 9 | 8 | 0 | 0 |
| | | Post | 9 | 9 | 9 | 9 | 9 | 9 | 9 | 9 | 9 | 9 | 9 | 9 | 9 | 9 | 9 | - | 9 | 9 | 6 | 9 | 9 | 9 | 9 | 9 | 5 |
| 81 | 1 | Pre | 9 | 9 | 5 | 2 | 4 | 5 | 5 | - | 9 | - | 9 | 9 | 9 | 9 | 9 | 5 | - | 0 | 1 | - | 9 | 9 | 9 | 8 | 1 |
| | | Post | 9 | 9 | 9 | 9 | 9 | 7 | 8 | 9 | 9 | 9 | 9 | 9 | 9 | 9 | 9 | - | 9 | 9 | 9 | 8 | 9 | 9 | 9 | 9 | 7 |
| 82 | 0.0625 | Pre | 0 | 0 | 0 | 0 | 0 | 0 | 0 | 0 | 0 | 0 | 0 | 0 | 0 | 0 | 0 | - | - | 0 | 0 | - | 0 | 0 | 0 | 0 | 0 |
| | | Post | 7 | 9 | 5 | 3 | 8 | 3 | 5 | 9 | 7 | 8 | 9 | 7 | 9 | 9 | 7 | - | 9 | 0 | 2 | 3 | 5 | 2 | 3 | 9 | 1 |

TABLE VII

Abbreviations used for Test Plants

Sb - Sugar beet
Rp - Rape
Ct - Cotton
Sy - Soybean
Mz - Maize
Ww - Winter wheat
Rc - Rice
Bd - *Bidens pilosa*
Ip - *Ipomoea lacunosa* (pre-emergence)
*Ipomoea hederacea* (post-emergence)
Am - *Amaranthus retroflexus*
Pi - *Polygonm aviculare*
Ca - *Chenopodium album*
Ga - *Galium aparine*
Xa - *Xanthium spinosum*
Xs - *Xanthium strumarium*
Ab - *Abutilon theophrasti*
Eh - *Euphorbia heterophylla*
Av - *Avena fatua*
Dg - *Digitaria sanguinalis*
Al - *Alopecurus myosuroides*
St - *Setaria viridis*
Ec - *Echinochloa crus-galli*
Sh - *Sorghum halepense*
Ag - *Agropyron repens*
Ce - *Cyperus esculentes*

## Claims

1. A compound of formula (I):

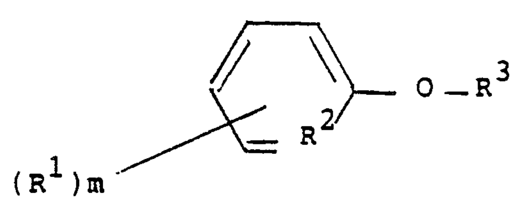

(I)

in which $R^1$ is independently selected from H, CN, $NO_2$, halogen, lower alkyl, lower haloalkyl; m is an integer of from 1 to 4; $R^2$ is N or $CR^1$ where $R^1$ is as defined above; $R^3$ is a group of formula (a), (b) or (c):

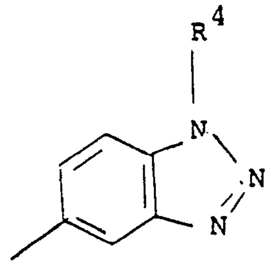

(a)

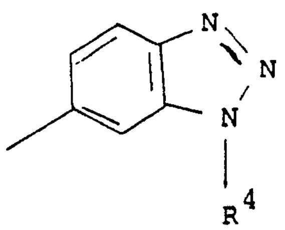

(b)

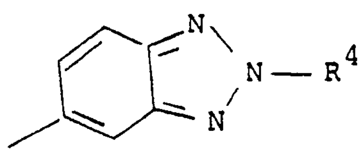

(c)

or N-oxide or quaternary salt derivatives thereof wherein $R^4$ is a group

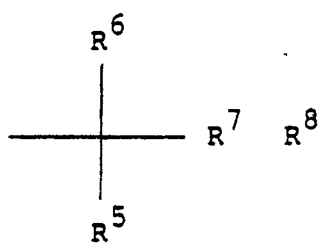

where $R^5$, $R^6$ are selected independently from H, lower alkyl, halogen or $R^5$ and $R^6$ together form $=CH_2$ or $R^5$, $R^6$ and the carbon atom to which they are attached together form a $C_{3-6}$ cycloalkyl ring;
$R^7$ is $(CH_2)_n$,

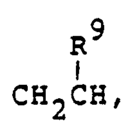

CH=CH,
$\overset{O}{\overset{\|}{C}}CH_2$, $CHOHCH_2$ or $CHOR^{10}CH_2$ where $R^9$ is lower alkyl and $R^{10}$ is lower alkyl or phenyl; and n is 0, 1 or 2;
$R^8$ is $COOR^{11}$, OH, $OR^{10}$,

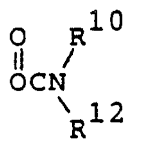, 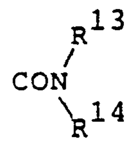,

COSR$^{11}$ CHO, CN, CH = NOR$^{12}$,

$\overset{O}{\overset{\|}{P}}$ $(OR^{12})_2$, $OSO_3H$, $SO_3H$; where

$R^{10}$ is as defined hereinbefore;

$R^{11}$ is hydrogen, optionally substituted lower alkyl, optionally substituted lower alkenyl, optionally substituted lower alkynyl, $[(CH_2)_2O]_p$ $(CH_2)_q$ $OR^{10}$, $-N = C(R^{10})_2$, optionally substituted phenyl, optionally substituted benzyl, optionally substituted cycloalkyl; p is 0, 1 or 2; q is an integer from 2 to 5 inclusive;

$R^{12}$ is H or lower alkyl;

$R^{13}$ and $R^{14}$ are selected independently from H, lower alkyl, lower cycloalkyl, alkenyl, alkynyl, $SO_2R^{10}$, $N(R^{12})_2$, $\overset{+}{N}(R^{10})_3$; additionally $R^{14}$ may be an optionally hydroxy- or phenyl-substituted alkyl radical of 1 to 4 carbon atoms, a phenyl or chlorophenyl radical, an alkoxy radical of 1 to 4 carbon atoms, or a group $-NR^{15}R^{16}$ wherein $R^{15}$ is hydrogen or alkyl of 1 to 4 carbon atoms and $R^{16}$ is hydrogen, alkyl of 1 to 4 carbon atoms, phenyl, or chlorophenyl, or the group $-NR^{13}R^{14}$ constitutes a 5 or 6-membered heterocyclic ring or alkyl quaternary derivatives; and, in the case of compounds wherein $R^8$ is a carboxyl group, salts thereof.

2. A compound of formula (I) substantially in the form of the (R) enantiomer.

3. A compound according to claims 1 or 2 wherein $R^3$ is

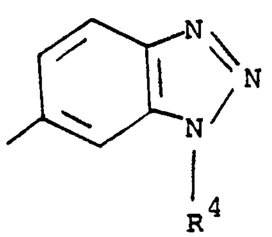 or 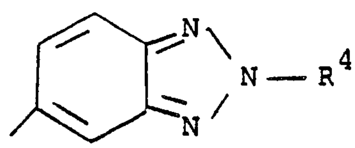,

4. A compound according to any of the preceding claims where $R^4$ is

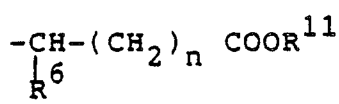

where n, $R^6$ and $R^{11}$ are as defined in claim 1.

5. A compound according to claim 4 wherein $R^{11}$ is $C_{1-4}$ alkyl and $R^6$ is H or $C_{1-4}$ alkyl.

6. A herbicidal composition comprising a compound of formula (I) as defined in claim 1 in combination with a carrier or diluent.

7. A method of killing or controlling the growth or unwanted plants which method comprises applying to the plants or to a locus thereof an effective amount of a compound of formula (I) as defined in claim 1.

8. A process for preparing a compound of formula (I) as defined in claim 1 which comprises:

(a) reacting a compound of formula (II)

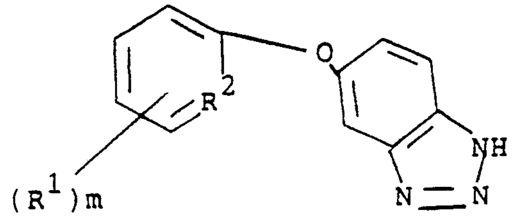

(II)

wherein $R^1$, $R^2$ and m are as defined in claim 1 with a compound of formula (III)

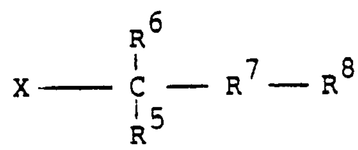

wherein $R^5$, $R^6$, $R^7$, $R^8$ are as defined in relation to formula (I) and X is a leaving group in the presence of a base or with a compound of formula (IV)

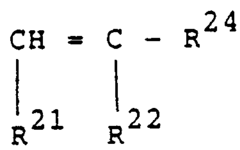

(IV)

wherein $R^{21}$ and $R^{22}$ are independently H or lower alkyl and $R^{23}$ is CN, CHO or $COOR^{24}$ where $R^{24}$ is lower alkyl in the presence of a base; or

b) where $R^3$ is a sub-group (b) and $R^4$ is a group $R^{25}$-CH-$R^{26}$, wherein $R^{25}$ is $CH_2OH$, COOH, $COO^-M^+$, $COOR^{29}$ or

O

P $(OC_2H_5)_2$ where $M^+$ is a cation, $R^{29}$ is lower alkyl and $R^{26}$ is H or lower alkyl, cyclisation of a compound of formula (V):

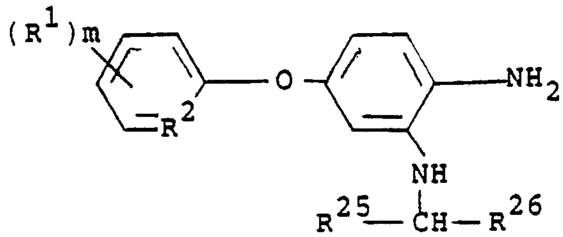

(V)

c) where $R^3$ is a sub-group (a) or (b), reaction of a compound of formula (VIII)

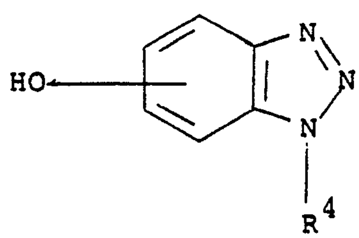

(VIII)

where $R^4$ is as defined in relation to formula (I) with a compound of formula (VII)

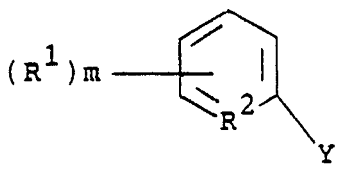

(VII)

where R', $R^2$ and m are as defined in relation to formula (I) and Y is halo; or

d) where $R^3$ is sub-group (b) and $R^4$ is

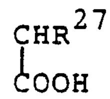

where $R^{27}$ is H or lower alkyl, reaction of a compound of formula (VI)

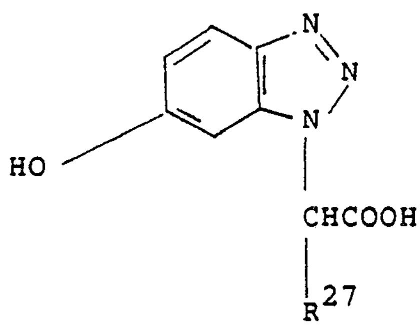

(VI)

with a compound of formula (VII) and thereafter if desired or necessary carrying out one or more of the following steps:

i) when $R^8$ of formula (I) is alkoxycarbonyl hydrolysing to the corresponding acid.

ii) when $R^8$ of formula (I) is COOH esterifying or forming a salt, amide, sulphonamide, hydrazide or hydrazinium derivative.

iii) when $R^8$ of formula (I) is an alcohol, oxidation to COOH or CHO.

9. A compound of formula (V):

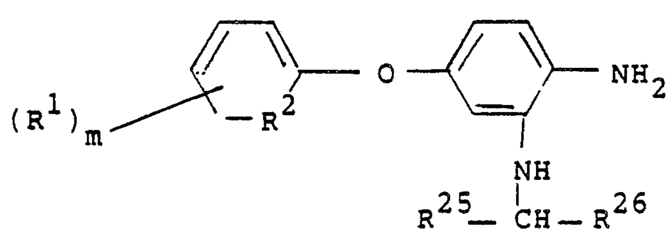

(V)

where $(R^1)_m$, $R^2$, $R^{25}$ and $R^{26}$ are as defined in claim 8.

10. A compound of formula (II):

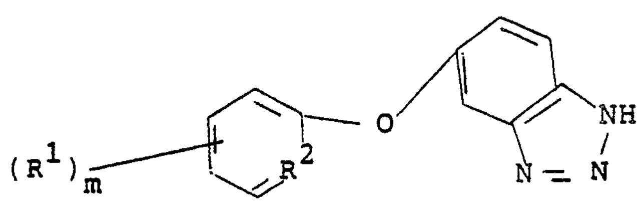

(II)

wherein $(R^1)_m$ and $R^2$ are as defined in claim 8 provided that (II) is not 5-(2-chloro-4-(trifluoromethyl)-

phenoxy]-1H-benzotriazole.

11. A compound of formula (VIIIA):

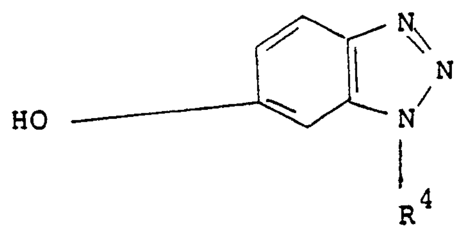

(VIIIA)

where $R^4$ is as defined in claim 8.